(19) Europäisches Patentamt
European Patent Office
Office européen des brevets

(11) Veröffentlichungsnummer: **0 136 266**
**A2**

# (12) EUROPÄISCHE PATENTANMELDUNG

(21) Anmeldenummer: **84810454.3**

(22) Anmeldetag: **17.09.84**

(51) Int. Cl.⁴: **C 07 D 501/20**, A 61 K 31/545

(30) Priorität: **22.09.83 CH 5154/83**

(43) Veröffentlichungstag der Anmeldung: **03.04.85 Patentblatt 85/14**

(84) Benannte Vertragsstaaten: **AT BE CH DE FR GB IT LI LU NL SE**

(71) Anmelder: **CIBA-GEIGY AG, Postfach, CH-4002 Basel (CH)**

(72) Erfinder: **Fechtig, Bruno, Dr., Hinterlindenweg 1, CH-4153 Reinach (CH)**

(54) **7beta-Acylamido-3-cephem-4-carbonsäureester, Verfahren zu ihrer Herstellung, pharmazeutische Präparate, welche diese Verbindungen enthalten, und Verwendung von letzteren.**

(57) 7β-Acylamido-3-cephem-4-carbonsäureester der Formel

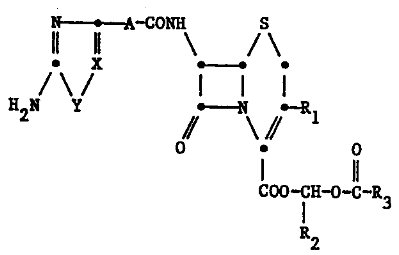

(I)

worin X Stickstoff oder die Gruppe $\overset{\|}{-C-H}$, Y Sauerstoff, Schwefel oder die Gruppen $>N-H$ oder $>N$-Niederalkyl, A Methylen oder durch Amino, substituiertes Amino, Hydroxy, Carboxy, Hydroyimino oder veräthertes oder verestertes hydroxyimino substituiertes Methylen, $R_1$ Wasserstoff, Halogen, Niederalkyl oder Niederalkoxy, $R_2$ Wasserstoff, Niederalkyl, Phenyl oder substituiertes Phenyl und $R_3$ unsubstituiertes oder substituiertes, aromatisches, fünf- oder sechsgliedriges Heterocyclyl, Phenyl oder substituiertes Phenyl bedeuten, Stereoisomere, Mischungen von diesen Stereoisomeren und ihre Salze besitzen antibiotische Eigenschaften und sind gegen grampositive und gramnegative Erreger wirksam. Die neuen Verbindungen eignen sich für die perorale Applikation und können z.B. in Form von antibiotisch wirksamen Präparaten zur Behandlung von Infektionen verwendet werden. Die neuen Verbindungen werden in an sich bekannter Weise aus zum Teil neuen Zwischenprodukten hergestellt.

EP 0 136 266 A2

ACTORUM AG

CIBA-GEIGY AG
Basel (Schweiz)

4-14590/+

# 7ß-Acylamido-3-cephem-4-carbonsäureester, Verfahren zu ihrer Herstellung, pharmazeutische Präparate, welche diese Verbindungen enthalten, und Verwendung von letzteren

Die vorliegende Erfindung betrifft neue, physiologisch spaltbare 7ß-Acylamido-3-cephem-4-carbonsäureester, Verfahren zu ihrer Herstellung, pharmazeutische Präparate, welche diese Ester enthalten, und die Verwendung dieser Ester als Arzneimittel und zur Herstellung von pharmazeutischen Präparaten.

Die vorliegende Erfindung betrifft 7ß-Acylamido-3-cephem-4-carbonsäureester der Formel:

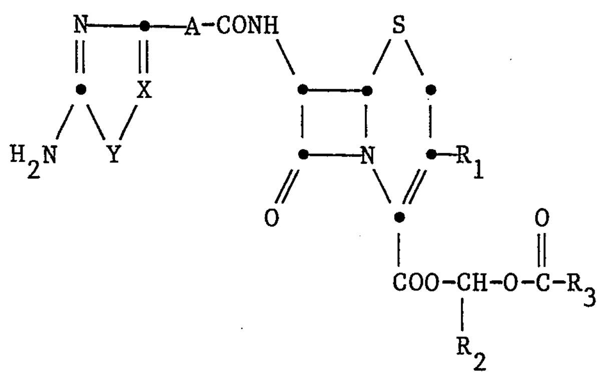

(I),

worin X Stickstoff oder die Gruppe $-\overset{\|}{C}-H$, Y Sauerstoff, Schwefel oder die Gruppen >N-H oder >N-Niederalkyl, A Methylen oder durch Amino, substituiertes Amino, Hydroxy, Carboxy, Hydroxyimino oder veräthertes oder verestertes Hydroxyimino substituiertes Methylen, $R_1$ Wasserstoff, Halogen, Niederalkyl oder Niederalkoxy, $R_2$ Wasserstoff, Niederalkyl, Phenyl oder substituiertes Phenyl und $R_3$ unsubstituiertes oder substituiertes, aromatisches, fünf- oder sechsgliedriges

Heterocyclyl, Phenyl oder substituiertes Phenyl bedeuten, Stereoisomere, Mischungen von diesen Stereoisomeren, Salze, Verfahren zur Herstellung dieser Ester, pharmazeutische Präparate, welche diese Ester enthalten, und die Verwendung dieser Ester als Arzneimittel oder zur Herstellung von pharmazeutischen Präparaten.

In der Beschreibung der vorliegenden Erfindung bedeutet der im Zusammenhang mit Gruppen oder Resten, z.B. Niederalkyl, Niederalkylen, Niederalkoxy, Niederalkanoyl etc. verwendete Ausdruck "Nieder", dass die so bezeichneten Gruppen oder Reste, falls nicht ausdrücklich anders definiert, bis zu 7 und bevorzugt bis zu 4 C-Atome enthalten.

Wenn A durch Amino, substituiertes Amino, Hydroxy oder Carboxy substituiertes Methylen bedeutet, hat das C-Atom der substituierten Methylengruppe A (2-C-Atom der 7ß-Seitenkette) die R- oder die S-Konfiguration. Die vorliegende Erfindung umfasst die reinen R- und S-Verbindungen in Bezug auf dieses C-Atom, sowie R-, S-Gemische von Verbindungen der Formel I.

Wenn A durch Hydroxyimino oder veräthertes oder verestertes Hydroxyimino substituiertes Methylen bedeutet, kann die freie, verätherte oder veresterte Hydroxygruppe am doppelt gebundenen Stickstoff in syn- (oder Z-) oder anti- (oder E-) Stellung stehen, wobei die syn- (oder Z-) Stellung bevorzugt ist. In der syn-Stellung ist die freie, verätherte oder veresterte Hydroxygruppe zum Cephemgerüst und in anti-Stellung entgegengesetzt gerichtet.

Wenn $R_2$ Niederalkyl, Phenyl oder substituiertes Phenyl bedeutet, hat das durch $R_2$ substituierte C-Atom die R- oder die S-Konfiguration. Die vorliegende Erfindung umfasst die reinen R- und S-Verbindungen in Bezug auf dieses C-Atom und die R-, S-Gemische von Verbindungen der Formel I.

Die vorliegende Erfindung umfasst sämtliche tautomeren Formen, welche den Verbindungen der Formel I entsprechen, auch wenn in der Beschreibung nur bestimmte tautomere Formen erwähnt sein sollten.

Die in der Beschreibung der vorliegenden Erfindung verwendeten allgemeinen Ausdrücke haben die folgenden Bedeutungen:

Y mit der Bedeutung >N-Niederalkyl ist vorzugsweise >N- Methyl, ferner >N-Aethyl.

Substituiertes Amino ist z.B. Niederalkylamino, vorzugsweise Methylamino, ferner Aethyl-, n-Propyl oder tert-Butylamino, Diniederalkylamino, z.B. Dimethylamino, Niederalkanoylamino, vorzugsweise Formylamino, ferner Acetylamino, N-Niederalkyl-N-niederalkanoylamino, z.B. N-Methyl-N-formylamino oder N-Methyl-N-acetylamino, Niederalkylsulfonylamino, vorzugsweise Methylsulfonylamino (Mesylamino), ferner Aethylsulfonylamino, N-Niederalkyl-N-niederalkylsulfonylamino, z.B. N-Methyl-N-methylsulfonylamino, Aminoniederalkylsulfonylamino, z.B. 2-Aminoäthylsulfonylamino (Taurylamino), oder N-Niederalkyl-N-aminoniederalkylsulfonylamino, z.B. N-Methyl-N-2-aminoäthylsulfonylamino.

Veräthertes Hydroxyimino ist z.B. Niederalkoxyimino, vorzugsweise Methoxyimno, ferner Aethoxy-, n-Propoxy oder tert-Butoxyimino, Cycloalkoxyimino, vorzugsweise Cyclopropoxyimino, ferner Cyclobutoxy-, Cyclopentyloxy- oder Cyclohexyloxyimino, Niederalkenyloxyimino, z.B. Vinyl- oder Allyloxyimino, oder Carboxyniederalkoxyimino, z.B. 1- oder 2-Carboxyprop-2-yloxyimino, ferner Carboxymethoxyimino.

Verestertes Hydroxyimino ist z.B. Carbamoyloxyimino, Niederalkylcarbamoyloxyimino, z.B. Methylcarbamoyloxyimino, oder Diniederalkylcarbamoyloxyimino, z.B. Dimethylcarbamoyloxyimino.

Halogen $R_1$ ist bevorzugt Chlor, ferner Fluor oder Brom.

Niederalkyl $R_1$ hat 1-4 C-Atome und ist vorzugsweise Methyl, ferner Aethyl, n-Propyl oder n-Butyl.

Niederalkoxy $R_1$ hat 1-4 C-Atome und ist vorzugsweise Methoxy, ferner Aethoxy, n-Propoxy oder n-Butoxy.

Niederalkyl $R_2$ ist bevorzugt Methyl, ferner Aethyl.

Der Phenylrest $R_2$ oder $R_3$ kann durch einen bis drei Substituenten aus der Gruppe Niederalkyl, z.B. Methyl, Hydroxy, Niederalkoxy, z.B. Methoxy, Niederalkanoyl, z.B. Formyl oder Acetyl, Niederalkanoyloxy, z.B. Acetoxy, Halogen, z.B. Fluor, Chlor oder Brom, Nitro, Cyan, Amino, Niederalkylamino, z.B. Methylamino, Diniederalkylamino, z.B. Dimethylamino, Niederalkanoylamino, z.B. Acetylamino, Carbamoyl, Niederalkylcarbamoyl, z.B. Methylcarbamoyl, Diniederalkylcarbamoyl, z.B. Dimethylcarbamoyl, Niederalkylsulfonylamino, z.B. Methyl- oder Aethylsulfonylamino, Sulfamoyl, Niederalkylsulfamoyl, z.B. Methylsulfamoyl, Diniederalkylsulfamoyl, z.B. Dimethylsulfamoyl oder Di-n-propylsulfamoyl, Halogenniederalkyl, z.B. Chlormethyl oder Trifluormethyl, Hydroxyniederalkyl, z.B. Hydroxymethyl, und Aminoniederalkyl, z.B. Aminomethyl oder 2-Aminoäthyl, substituiert sein.

Substituiertes Phenyl $R_2$ oder $R_3$ ist vorzugsweise Niederalkyl-, Diniederalkyl- oder Triniederalkylphenyl, z.B. 4-Methyl-, 4-tert-Butyl-, 3,4-Dimethyl-, 2,3-Dimethyl-, 2,4-Dimethyl- oder 2,4,6-Trimethylphenyl, Hydroxy-, Dihydroxy- oder Trihydroxyphenyl, z.B. 4-Hydroxy-, 2,4-Dihydroxy-, 2,3,4-Trihydroxy-, 2,4,6-Trihydroxy-oder 3,4,5-Trihydroxyphenyl, Hydroxy-niederalkylphenyl, z.B. 2-Hydroxy-3-methylphenyl, Niederalkoxy-, Diniederalkoxy- oder Triniederalkoxyphenyl, z.B. 4-Methoxy-, 3,4-Dimethoxy-, 2,3-Dimethoxy-, 2,4-Dimethoxy- oder 3,4,5-Trimethoxyphenyl, Hydroxy-niederalkoxyphenyl, z.B. 4-Hydroxy-3-methoxyphenyl, Hydroxy-diniederalkoxyphenyl, z.B. 3-Hydroxy-2,4-dimethoxyphenyl, Niederalkanoylphenyl, z.B. 2-, 3- oder 4-Acetylphenyl, Niederalkanoyloxyphenyl, z.B. 2-, 3-, 4- oder

5-Acetoxyphenyl oder 3,4- oder 3,5-Diacetoxyphenyl, Halogenphenyl, z.B. 2-, 3- oder 4-Fluor- oder 2-, 3- oder 4-Chlorphenyl, Nitrophenyl, z.B. 2-, 3-oder 4-Nitrophenyl, Cyanphenyl, z.B. 2-, 3- oder 4-Cyanphenyl, Amino- oder Diaminophenyl, z.B. 2-, 3- oder 4-Aminophenyl oder 3,5-Diaminophenyl, Amino-hydroxyphenyl, z.B. 3-Amino-4-hydroxy- oder 4-Amino-2-hydroxyphenyl, Niederalkylaminophenyl, z.B. 2- oder 4-Methylaminophenyl, Diniederalkylaminophenyl, z.B. 2-, 3- oder 4-Dimethylaminophenyl, Niederalkanoylaminophenyl, z.B. 2-, 3- oder 4-Acetylaminophenyl, Sulfamoylphenyl, z.B. 2-, 3- oder 4-Sulfamoylphenyl, Halogenniederalkylphenyl, z.B. 2-, 3- oder 4-Trifluormethylphenyl, oder Aminoniederalkylphenyl, z.B. 2-, 3- oder 4-Aminomethylphenyl.

Ein aromatischer, fünf- oder sechsgliedriger, monocyclischer Heterocyclus $R_3$ hat mindestens ein C-Atom und ist mit einem seiner C-Atome mit der Carbonylgruppe verbunden und enthält 1-4 Stickstoffatome oder ein Sauerstoff- oder Schwefelatom und gegebenenfalls 1-3 Stickstoffatome.

Substituenten dieses Heterocyclus $R_3$ sind beispielsweise Niederalkyl, z.B. Methyl, Hydroxy, Niederalkoxy, z.B. Methoxy, Niederalkanoyloxy, z.B. Acetoxy, Halogen, z.B. Chlor, Amino, Mononiederalkylamino, z.B. Methylamino, Diniederalkylamino, z.B. Dimethylamino, Niederalkanoylamino, z.B. Acetylamino, Oxo oder Oxido.

Unsubstituiertes oder substituiertes, aromatisches, fünfgliedriges Heterocyclyl $R_3$ ist beispielsweise unsubstituiertes oder durch die genannten Substituenten substituiertes Aza-, Diaza-, Triaza-, Tetraaza-, Thia-, Thiaza-, Thiadiaza- oder Oxacyclyl, vorzugsweise Pyrrolyl, z.B. Pyrrol-2-yl, Niederalkylpyrrolyl, z.B. 1-Methylpyrrol-2-yl, Imidazolyl, z.B. Imidazol-2-yl, Niederalkylimidazolyl, z.B. 1-Methylimidazol-2-yl, Triazolyl, z.B. 1H-1,2,3-Triazol-5-yl, Niederalkyltriazolyl, z.B. 1-Methyl-1H-1,2,3-triazol-5-yl, Tetrazolyl, z.B. 1H-Tetrazol-5-yl, Niederalkyltetrazolyl, z.B. 1-Methyl-1H-tetrazol-5-yl, Thienyl, z.B. Thien-3- oder -2-yl, Thiazolyl, z.B. Thiazol-2-yl, Thiadiazolyl, z.B. 1,2,3-Thiadiazol-5-yl oder Furyl, z.B. Fur-2- oder -3-yl.

Unsubstituiertes oder substituiertes, aromatisches, sechsgliedriges Heterocyclyl $R_3$ ist beispielsweise unsubstituiertes oder durch die genannten Substituenten substituiertes Aza-, Diaza- oder Oxacyclyl, vorzugsweise Pyridyl, z.B. Pyrid-2-, -3- oder -4-yl, Hydroxypyridyl, z.B. 2-Hydroxypyrid-5-yl, Dihydroxypyridyl, z.B. 2,6-Dihydroxypyrid-4-yl, Aminopyridyl, z.B. 2-Aminopyrid-3-yl, Dihydroxypyrimidinyl, z.B. 2,4-Dihydroxypyrimidin-6-yl, Pyrazinyl, z.B. Pyrazin-2-yl, Aminopyrazin-2-yl, z.B. 3-Aminopyrazin-2-yl oder Pyronyl, z.B. Pyr-2-on-5-yl.

In Verbindungen der Formel I vorhandene funktionelle Gruppen, insbesondere die Carboxy-, Amino- und Hydroxygruppe sind gegebenenfalls durch geeignete Schutzgruppen (conventional protecting groups) geschützt, die üblicherweise bei der Synthese von Penicillin-, Cephalosporin- und Peptid-Verbindungen verwendet werden. Diese Schutzgruppen sollen die betreffenden funktionellen Gruppen gegen unerwünschte Nebenreaktionen wie Acylierungen, Verätherungen, Veresterungen, Oxydationen, Solvolyse etc. schützen und unter schonenden Reaktionsbedingungen leicht abspaltbar sein, z.B. solvolytisch, reduktiv, photolytisch oder auch enzymatisch.

Der Schutz von funktionellen Gruppen mit solchen Schutzgruppen, die Schutzgruppen selbst, sowie ihre Abspaltungsreaktionen, sind beispielsweise in "Protective Groups in Organic Chemistry", Plenum Press, London und New York 1973, in Greene, Th.W., "Protective Groups in Organic Synthesis", Wiley, New York 1981, in "The Peptides", Vol. I, Schröder and Lubke, Academic Press, London und New York 1965, sowie in "Methoden der organischen Chemie", Houben-Weyl, 4. Auflage, Bd. 15/I, Georg Thieme Verlag, Stuttgart 1974, beschrieben.

Eine Carboxygruppe ist üblicherweise in veresterter Form geschützt, wobei die Estergruppe unter schonenden Bedingungen selektiv spaltbar ist. Eine in veresterter Form geschützte Carboxygruppe ist in erster Linie durch eine Niederalkylgruppe verestert, welche in 1-Stellung der Niederalkylgruppe verzweigt oder in 1- oder 2-Stellung der Niederalkylgruppe durch geeignete Substituenten substituiert ist.

Eine geschützte Carboxygruppe, welche durch eine Niederalkylgruppe verestert ist, die in 1-Stellung der Niederalkylgruppe verzweigt ist, ist beispielsweise tert-Niederalkoxycarbonyl, z.B. tert-Butoxycarbonyl, Arylmethoxycarbonyl mit einem oder zwei Arylresten, worin Aryl vorzugsweise unsubstituiertes oder z.B. durch Niederalkyl, z.B. tert-Niederalkyl, z.B. tert-Butyl, Niederalkoxy, z.B. Methoxy, Hydroxy, Halogen, z.B. Chlor, und/oder Nitro mono-, di- oder trisubstituiertes Phenyl bedeutet, beispielsweise Benzyloxycarbonyl, durch die genannten Substituenten substituiertes Benzyloxycarbonyl, z.B. 4-Nitrobenzyloxycarbonyl oder 4-Methoxybenzyloxycarbonyl, Diphenylmethoxycarbonyl oder die genannten Substituenten substituiertes Diphenylmethoxycarbonyl, z.B. Di-(4-methoxyphenyl)-methoxycarbonyl.

Eine geschützte Carboxygruppe, welche durch eine Niederalkylgruppe verestert ist, welche in 1- oder 2-Stellung der Niederalkylgruppe durch geeignete Substituenten substituiert ist, ist beispielsweise 1-Niederalkoxyniederalkoxycarbonyl, z.B. Methoxymethoxycarbonyl, 1-Methoxyäthoxycarbonyl oder 1-Aethoxymethoxycarbonyl, 1-Niederalkylthioniederalkoxycarbonyl, z.B. 1-Methylthiomethoxycarbonyl oder 1-Aethylthioäthoxycarbonyl, Aroylmethoxycarbonyl, z.B. Phenacyloxycarbonyl, sowie 2-Halogenniederalkoxycarbonyl, z.B. 2,2,2-Trichloräthoxycarboyl, 2-Bromäthoxycarbonyl oder 2-Jodäthoxycarbonyl.

Eine Carboxygruppe kann auch als organische Silyloxycarbonylgruppe geschützt sein. Eine organische Silyloxycarbonylgruppe ist beispielsweise eine Triniederalkylsilyloxycarbonylgruppe, z.B. Trimethylsilyloxycarbonyl. Das Siliciumatom der Silyloxycarbonylgruppe kann auch durch zwei Niederalkylgruppen, z.B. Methylgruppen, und die

Aminogruppe oder die zur Carboxygruppe gehörende Hydroxygruppe eines zweiten Moleküls der Formel I substituiert sein. Verbindungen mit solchen Schutzgruppen lassen sich z.B. bei Verwendung von Dimethyldichlorsilan als Silylierungsmittel herstellen.

Eine geschützte Carboxygruppe ist bevorzugt tert-Niederalkoxycarbonyl, z.B. tert-Butoxycarbonyl, Benzyloxycarbonyl, 4-Nitrobenzyloxycarbonyl und Diphenylmethoxycarbonyl.

Eine geschützte Carboxygruppe ist ausserdem unter physiologischen Bedinungen, z.B. enzymatisch, spaltbares, verestertes Carboxy, in erster Linie Acyloxyniederalkoxycarbonyl, worin Acyl z.B. die Acylgruppe einer Carbonsäure, eines Kohlensäuremonoesters oder einer Aminosäure darstellt oder worin Acyloxyniederalkoxy, z.B. Acyloxymethoxy, den Rest eines Lactons bildet.

Acyl ist vorzugsweise die Acylgruppe einer gegebenenfalls verzweigten Niederalkancarbonsäure, die Acylgruppe der durch gegebenenfalls durch verzweigtes Niederalkyl monoveresterten Kohlensäure oder die Acylgruppe einer gegebenenfalls verweigten α-Aminoniederalkancarbonsäure.

Unter physiologischen Bedingungen, z.B. enzymatisch, spaltbares, verestertes Carboxy ist vorzugsweise Niederalkanoyloxyniederalkoxycarbonyl, z.B. Niederalkanoyloxymethoxycarbonyl oder Niederalkanoyloxyäthoxycarbonyl, z.B. Acetoxymethoxycarbonyl, Pivaloyloxymethoxycarbonyl oder 1-Propionyloxyäthoxycarbonyl, Niederalkoxycarbonyloxyniederalkoxycarbonyl, z.B. 1-Aethoxycarbonyloxyäthoxycarbonyl oder tert-Butoxycarbonyloxymethoxycarbonyl, Aminoniederalkanoyloxymethoxycarbonyl, insbesondere α-Aminoniederalkanoyloxymethoxycarbonyl, z.B. Glycyloxymethoxycarbonyl, L-Valyloxymethoxycarbonyl oder L-Leucyloxymethoxycarbonyl, ferner Phthalidyloxycarbonyl, z.B. 2-Phthalidyloxycarbonyl, oder Indanyloxycarbonyl, z.B. 5-Indanyloxycarbonyl.

Eine Aminogruppe kann z.B. in Form einer unter schonenden Bedingungen selektiv spaltbaren Acylamino-, verätherten Mercaptoamino- oder Silylaminogruppe oder als Azidogruppe geschützt sein.

In einer leicht spaltbaren Acylaminogruppe ist Acyl beispielsweise die Acylgruppe einer organischen Carbonsäure mit bis zu 10 Kohlenstoffatomen, insbesondere einer unsubstituierten oder einer, z.B. durch Halogen oder Aryl, substituierten Niederalkancarbonsäure oder der unsubstituierten oder, z.B. durch Halogen, Niederalkoxy oder Nitro, substituierten Benzoesäure oder eines Kohlensäurehalbesters. Eine solche Acylgruppe ist beispielsweise Halogenniederalkanoyl, z.B. 2-Halogenacetyl, insbesondere 2-Chlor-, 2-Brom-, 2-Jod-, 2,2,2-Trifluor- oder 2,2,2-Trichloracetyl, Benzoyl oder z.B. durch Halogen, z.B. Chlor, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung durch geeignete Substituenten substituiertes Niederalkoxycarbonyl.

In 1-Stellung des Niederalkylrestes verzweigtes Niederalkoxycarbonyl ist beispielsweise tert-Niederalkoxycarbonyl, z.B. tert-Butoxycarbonyl (BOC), Arylmethoxycarbonyl mit einem oder zwei Arylresten, worin Aryl vorzugsweise unsubstituiertes oder z.B. durch Niederalkyl, insbesondere tert-Niederalkyl, z.B. tert-Butyl, Niederalkoxy, z.B. Methoxy, Hydroxy, Halogen, z.B. Chlor, und/oder Nitro, mono-, di-oder trisubstituiertes Phenyl bedeutet, beispielsweise Diphenylmethoxycarbonyl oder Di-(4-methoxyphenyl)-methoxycarbonyl.

In 1- oder 2-Stellung durch geeignete Substituenten substituiertes Niederalkoxycarbonyl ist beispielsweise Aroylmethoxycarbonyl, z.B. Phenacyloxycarbonyl, 2-Halogenniederalkoxycarbonyl, z.B. 2,2,2-Trichloräthoxycarbonyl, 2-Bromäthoxycarbonyl oder 2-Jodäthoxycarbonyl, oder 2-(trisubstituiertes Silyl)-äthoxycarbonyl, worin die Silylgruppe durch organische Reste, z.B. Niederalkyl, Phenylniederalkyl oder Phenyl substituiert ist, beispielsweise 2-Triniederalkylsilyl-

äthoxycarbonyl, z.B. 2-Trimethylsilyläthoxycarbonyl oder 2-(Di-n-butylmethylsilyl)-äthoxycarbonyl, oder 2-Triphenylsilyläthoxycarbonyl.

In einer verätherten Mercaptoaminogruppe ist die verätherte Mercaptogruppe in erster Linie Arylthio, z.B. 4-Nitrophenylthio.

Eine Silylaminogruppe ist beispielsweise eine Triniederalkylsilylaminogruppe, z.B. Trimethylsilylamino. Das Siliciumatom der Silylaminogruppe kann auch nur durch zwei Niederalkylgruppen, z.B. Methylgruppen, und die Aminogruppe oder Carboxylgruppe eines zweiten Moleküls der Formel I substituiert sein. Verbindungen mit solchen Schutzgruppen lassen sich z.B. bei Verwendung von Dimethyldichlorsilan als Silylierungsmittel herstellen.

Eine Aminogruppe kann auch in protonierter Form geschützt sein. Als Anionen sind in erster Linie die Anionen von starken anorganischen Säuren, wie von Halogenwasserstoffsäuren, z.B. das Chlor- oder Bromanion, oder von organischen Sulfonsäuren, wie p-Toluolsulfonsäure, geeignet.

Eine geschützte Aminogruppe ist bevorzugt tert-Butoxycarbonylamino (BOC), 4-Nitrobenzyloxycarbonylamino, Diphenylmethoxycarbonylaminio oder 2-Halogenniederalkoxycarbonylamino, z.B. 2,2,2-Trichloräthoxycarbonylamino.

Eine Hydroxygruppe kann beispielsweise durch eine Acylgruppe, z.B. durch Halogen, substituiertes Niederalkanoyl, z.B. 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2,2,2-Trichloräthoxycarbonyl, 4-Nitrobenzyloxycarbonyl, ein organischer Silylrest mit den weiter vorn genannten Substituenten, z.B. Trimethylsilyl, Methoxy, Hydroxy, Halogen, z.B. 2-Halogenniederalkoxycarbonylamino, z.B. 2,2,2-Trichloräthoxycarbonyl.

Eine Hydroxygruppe kann beispielsweise durch eine Acylgruppe, z.B. durch Halogen, substituiertes Niederalkanoyl, z.B. 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2,2,2-Trichloräthoxycarbonyl, 4-Nitrobenzyloxycarbonyl, ein organischer Silylrest mit den weiter vorn genannten Substituenten, z.B. Trimethylsilyl oder Dimethyl-n-butylsilyl, ferner eine leicht abspaltbare, veräthernde Gruppe, wie tert-Niederalkyl, z.B. tert-Butyl, ein oxa-oder ein thiaaliphatischer oder -cycloaliphatischer Kohlenwasserstoffrest, beispielsweise 1-Niederalkoxyniederalkyl oder 1-Niederalkylthioniederalkyl, z.B. Methoxymethyl, 1-Methoxyäthyl, 1-Aethoxyäthyl, Methylthiomethyl, 1-Methylthioäthyl oder 1-Aethylthioäthyl, oder 2-Oxa- oder 2-Thiacycloalkyl mit 5-7 Ringatomen, z.B. 2-Tetrahydrofuryl oder 2-Tetrahydropyranyl, oder ein entsprechendes Thiaanaloges, sowie 1-Phenyliederalkyl, z.B. Benzyl oder Diphenylmethyl, wobei die Phenylreste beispielsweise durch Halogen, z.B. Chlor, Niederalkoxy, z.B. Methoxy, und/oder Nitro substituiert sein können.

Salze sind in erster Linie die pharmazeutisch annehmbaren oder verwendbaren, nicht-toxischen Salze von Verbindungen der Formel I.

Solche Salze werden beispielsweise von einer in Verbindungen der Formel I vorhandenen sauren Gruppe, z.B. einer Carboxygruppe, gebildet und sind in erster Linie Metall- oder Ammoniumsalze, beispielsweise Alkalimetall- und Erdalkalimetall-, z.B. Natrium-, Kalium-, Magnesium- oder Calciumsalze, sowie Ammoniumsalze, welche mit Ammoniak oder geeigneten organischen Aminen gebildet werden, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung geeignet sind. Solche Basen sind beispielsweise Niederalkylamine, z.B. Triäthylamin, Hydroxyniederalkylamine, z.B. 2-Hydroxyäthylamin, Bis-(2-hydroxyäthyl)-amin oder Tris-(2-hydroxyäthyl)-amin, basische aliphatische Ester von Carbonsäuren, z.B.

4-Aminobenzoesäure-2-diäthyldiaminoäthylester, Niederalkylenamine, z.B. 1-Aethylpiperidin, Ccyloalkylamine, z.B. Dicyclohexylamin, oder Benzylamine, z.B. N,N'-Dibenzyläthylendiamin, ferner Basen vom Pyridintyp, z.B. Pyridin, Collidin oder Chinolin.

Die in Verbindungen der Formel I vorhandenen basischen Gruppen, z.B. Aminogruppen, können Säureadditionssalze, z.B. mit anorganischen Säuren, wie Salzsäure, Schwefelsäure oder Phosphorsäure, oder mit geeigneten organischen Carbon- oder Sulfonsäuren, z.B. ungesättigten Carbonsäuren, z.B. Fumarsäure oder Maleinsäure, Hydroxysäuren, z.B. Milchsäure, Weinsäure oder Citronensäure, oder einer aromatischen Säure, z.B. Salicylsäure, sowie mit Aminosäuren, wie Arginin und Lysin, bilden.

Zur Isolierung oder Reinigung können auch pharmazeutisch ungeeignete Salze Verwendung finden. Zur therapeutischen Anwendung gelangen aber nur pharmazeutisch verwendbare, nicht toxische Salze, die deshalb bevorzugt sind.

Die Verbindungen der Formel I und ihre pharmazeutisch annehmbaren, nicht-toxischen Salze sind wertvolle, antibiotisch wirksame Stoffe, die insbesondere als antibakterielle Antibiotika verwendet werden können. Beispielsweise sind Verbindungen der Formel I in vivo bei peroraler Applikation an der Maus gegen systemische Infektionen durch grampositive Keime, z.B. Streptococcus pyogenes, in einem Dosisbereich von ca. 0,5 bis 3,0 mg/kg ($ED_{50}$) oder gegen systemische Infektionen durch gramnegative Keime, z.B. Enterobakterien, z.B. Escherichia coli, in einem Dosisbereich von ca. 0,2 bis 3,0 mg/kg ($ED_{50}$) wirksam.

Die neuen Verbindungen der Formel I können daher als orale, antibakterielle Antibiotika, z.B. in Form von oral verabreichbaren, pharmazeutischen Präparaten, z.B. Kapseln, Tabletten, Sirupen oder dergleichen, zur Bekämpfung von Infektionen verwendet werden.

Die Verbindungen der Formel I zeichnen sich durch eine überraschend gute Resorption nach oraler Verabreichung aus, wie durch hohe renale Ausscheidung (RE) nachgewiesen wird. So wird bei peroraler Verabreichung z.B. des Hydrochlorids des erfindungsgemässen 7ß-[(2-(2-Aminothiazol-4-yl)-2-Z-methoxyiminoacetamido]-3-cephem-4-carbonsäure-2-acetoxybenzoyloxymethylesters an der Maus eine renale Ausscheidung von 23,2 % und an der Ratte eine renale Ausscheidung von 32,5% der verabreichten Menge des Wirkstoffs gemessen.

Die Erfindung hat vorzugsweise Verbindungen der Formel I zum Gegenstand, worin X Stickstoff oder die Gruppe $\overset{\|}{\text{-C-H}}$, Y Sauerstoff oder Schwefel, A durch Amino, substituiertes Amino, z.B. Niederalkanoylamino, z.B. Formylamino, Niederalkylsulfonylamino, z.B. Methylsulfonylamino, N-Niederalkyl-N-niederalkylsulfonylamino, z.B. N-Methyl-N-methylsulfonylamino, oder N-Aminoniederalkylsulfonyl-N-niederalkylamino, z.B. N-2-Aminoäthylsulfonyl-N-methylamino, Hydroxyimino oder veräthertes Hydroxyimino z.B. Niederalkoxyimino, z.B. Methoxyimino, oder Carboxyniederalkoxyimino, z.B. 2-Carboxy-2-propoxyimino, substituiertes Methylen, $R_1$ Wasserstoff, Halogen, z.B. Chlor, Niederalkyl, z.B. Methyl, oder Niederalkoxy, z.B. Methoxy, $R_2$ Wasserstoff, Niederalkyl, z.B. Methyl, Phenyl oder substituiertes Phenyl, z.B. Niederalkyl-, Diniederalkyl- oder Triniederalkylphenyl, z.B. 2-, 3- oder 4-Methyl-, 2-, 3- oder 4-tert-Butyl, 3,4-Dimethyl-, 2,3-Dimethyl-, 2,4-Dimethyl- oder 2,4,6-Trimethylphenyl, Hydroxy-, Dihydroxy- oder Trihydroxyphenyl, z.B. 4-Hydroxy-, 2,4-Dihydroxy-, 2,3,4-Trihydroxy-, 2,4,6-Trihydroxy- oder 3,4,5-Trihydroxyphenyl, Hydroxy-niederalkylphenyl, z.B. 2-Hydroxy-3-methylphenyl, Niederalkoxy-, Diniederalkoxy- oder Triniederalkoxyphenyl, z.B. 4-Methoxy-, 3,4-Dimethoxy-, 2,3-Dimethoxy-, 2,4-Dimethoxy- oder 3,4,5-Trimethoxyphenyl, Hydroxy-niederalkoxyphenyl, z.B. 4-Hydroxy-3-methoxyphenyl, Hydroxy-diniederalkoxyphenyl, z.B. 3-Hydroxy-2,4-dimethoxyphenyl, Niederalkanoylphenyl, z.B. 2-, 3- oder 4-Acetylphenyl, Niederalkanoyloxyphenyl, z.B. 2-, 3-, 4- oder 5-Acetoxyphenyl oder 3,4- oder 3,5-Diacetoxyphenyl, Halogenphenyl, z.B. 2-, 3- oder 4-Fluor- oder 2-, 3- oder

4-Chlorphenyl, Nitrophenyl, z.B. 2-, 3- oder 4-Nitrophenyl, Cyanphenyl, z.B. 2-, 3- oder 4-Cyanphenyl, Amino- oder Diaminophenyl, z.B. 2-, 3- oder 4-Aminophenyl oder 3,5-Diaminophenyl, Amino-hydroxyphenyl, z.B. 3-Amino-4-hydroxy-oder 4-Amino-2-hydroxyphenyl, Niederalkylaminophenyl, z.B. 2- oder 4-Methylaminophenyl, Diniederalkylaminophenyl, z.B. 2-, 3- oder 4-Dimethylaminophenyl, Niederalkanoylaminophenyl, z.B. 2-, 3- oder 4-Acetylaminophenyl, Sulfamoylphenyl, z.B. 2-, 3- oder 4-Sulfamoylphenyl, Halogenniederalkylphenyl, z.B. 2-, 3- oder 4-Trifluormethylphenyl, oder Aminoniederalkylphenyl, z.B. 2-, 3- oder 4-Aminomethylphenyl, und $R_3$ Pyrrolyl, z.B. Pyrrol-2-yl, Niederalkylpyrrolyl, z.B. 1-Methylpyrrol-2-yl, Imidazolyl, z.B. Imidazol-2-yl, Niederalkylimidazolyl, z.B. 1-Methylimidazol-2-yl, Triazolyl, z.B. 1H-1,2,3-Triazol-5-yl, Niederalkyltriazolyl, z.B. 1-Methyl-1H-1,2,3-triazol-5-yl, Tetrazolyl, z.B. 1H-Tetrazol-5-yl, Niederalkyltetrazolyl, z.B. 1-Methyl-1H-tetrazol-5-yl, Thienyl, z.B. Thien-3-oder -2-yl, Thiazolyl, z.B. Thiazol-2-yl, Thiadiazolyl, z.B. 1,2,3-Thiadiazol-5-yl, oder Furyl z.B. Fur-2-oder -3-yl, Pyrid-2-, -3- oder -4-yl, Hydroxypyridyl, z.B. 2-Hydroxypyrid-5-yl, Dihydroxypyridyl, z.B. 2,6-Dihydroxypyrid-4-yl, Aminopyridyl, z.B. 2-Aminopyrid-3-yl, Dihydroxypyrimidinyl, z.B. 2,4-Dihydroxypyrimidin-6-yl, Pyrazinyl, z.B. Pyrazin-2-yl, Aminopyrazin-2-yl, z.B. 3-Aminopyrazin-2-yl oder Pyronyl, z.B. Pyr-2-on-5-yl, Phenyl oder substituiertes Phenyl mit den unter $R_2$ genannten Substituenten bedeuten, und ihre pharmazeutisch annehmbaren Salze, z.B. die Hydrochloride.

Die Erfindung hat insbesondere Verbindungen der Formel I zum Gegenstand, worin X Stickstoff oder die Gruppe $-\overset{\|}{C}-H$, Y Sauerstoff oder Schwefel, A durch Hydroxyimino, veräthertes Hydroxyimino, z.B. Niederalkoxyimino, z.B. Methoxyimino, oder Carboxyniederalkoxyimino, z.B. 2-Carboxy-2-propoxyimino, substituiertes Methylen, $R_1$ Wasserstoff, Halogen, z.B. Chlor, oder Niederalkoxy, z.B. Methoxy, $R_2$ Wasserstoff, Niederalkyl, z.B. Methyl, Phenyl oder substituiertes Phenyl, z.B. Niederalkylphenyl, z.B. 2-, 3- oder 4-Methylphenyl, Hydroxy-, Dihydroxy-oder Trihydroxyphenyl, z.B. 4-Hydroxy-, 2,4-Dihydroxy-, 2,3,4-Trihydroxy-, 1,4,6-Trihydroxy- oder 3,4,5-Trihydroxyphenyl, Niederalkoxy-, Diniederalkoxy- oder Triniederalkoxyphenyl, z.B. 4-Methoxy-, 3,4-Dimethoxy-, 2,3-Dimethoxy-, 2,4-Dimethoxy- oder 3,4,5-Trimethoxyphenyl, Hydroxy-niederalkoxyphenyl, z.B. 4-Hydroxy-3-methoxyphenyl, Niederalkanoylphenyl, z.B. 2-, 3-oder 4-Acetylphenyl, Niederalkanoyloxyphenyl, z.B. 2-, 3-, 4- oder 5-Acetoxyphenyl, oder 3,4- oder 3,5-Diacetoxyphenyl, Halogenphenyl, z.B. 2-, 3- oder 4-Fluor- oder 2-, 3- oder 4-Chlorphenyl, Nitrophenyl, z.B. 2-, 3- oder 4-Nitrophenyl, Niederalkanoylaminophenyl, z.B. 2-, 3- oder 4-Acetylaminophenyl, Halogenniederalkylphenyl, z.B. 2-, 3- oder 4-Trifluormethylphenyl, oder Aminoniederalkylphenyl, z.B. 2-, 3- oder 4-Aminomethylphenyl, und $R_3$ Pyrrolyl, z.B. Pyrrol-2-yl, Niederalkylpyrrolyl, z.B. 1-Methylpyrrol-2-yl, Imidazolyl, z.B. Imidazol-2-yl, Niederalkylimidazolyl, z.B. 1-Methylimidazol-2-yl, Triazolyl, z.B. 1H-1,2,3-Triazol-5-yl, Niederalkyltriazolyl, z.B. 1-Methyl-1H-1,2,3-triazol-5-yl, Tetrazolyl, z.B. 1H-Tetrazol-5-yl, Niederalkyltetrazolyl, z.B. 1-Methyl-1H-tetrazol-5-yl, Thienyl, z.B. Thien-3- oder -2-yl, Thiazolyl, z.B. Thiazol-2-yl, Thiadiazolyl, z.B. 1,2,3-Thiadiazol-5-yl, oder Furyl, z.B. Fur-2- oder -3-yl, Pyrid-2-, -3- oder -4-yl, Hydroxypyridyl, z.B. 2-Hydroxypyrid-5-yl, Dihydroxypyridyl, z.B. 2,6-Dihydroxypyrid-4-yl, Aminopyridyl, z.B. 2-Aminopyrid-3-yl, Dihydroxypyrimidinyl, z.B. 2,4-Dihydroxypyrimidin-6-yl, Pyrazinyl, z.B. Pyrazin-2-yl, Aminopyrazinyl, z.B. 3-Aminopyrazin-2-yl oder Pyronyl, z.B. Pyr-2-on-5-yl, Phenyl oder substituiertes Phenyl, z.B. Niederalkyl-, Diniederalkyl- oder Triniederalkylphenyl, z.B. 2-, 3- oder 4-Methyl-, 2-, 3- oder 4-tert-Butyl-, 3,4-Dimethyl-, 2,3-Dimethyl-, 2,4-Dimethyl- oder 2,4,6-Trimethylphenyl, Hydroxy-, Dihydroxy- oder Trihydroxyphenyl, z.B. 4-Hydroxy-, 2,4-Dihydroxy-, 2,3,4-Trihydroxy-, 2,4,6-Trihydroxy- oder 3,4,5-Trihydroxyphenyl, Hydroxy-niederalkylphenyl, z.B. 2-Hydroxy-3-methylphenyl, Niederalkoxy-, Diniederalkoxy- oder Triniederalkoxyphenyl, z.B. 4-Methoxy-, 3,4-Dimethoxy-, 2,3-Dimethoxy-, 2,4-Dimethoxy- oder 3,4,5-Trimethoxyphenyl, Hydroxy-niederalkoxyphenyl, z.B. 4-Hydroxy-3-methoxyphenyl, Hydroxy-diniederalkoxyphenyl, z.B. 3-Hydroxy-2,4-dimethoxyphenyl, Niederalkanoylphenyl, z.B. 2-, 3- oder 4-Acetylphenyl, Niederalkanoyloxyphenyl, z.B. 2-,

3-, 4- oder 5-Acetoxyphenyl, oder 3,4- oder 3,5-Diacetoxyphenyl, Halogenphenyl, z.B. 2-, 3- oder 4-Fluor- oder 2-, 3- oder 4-Chlorphenyl, Nitrophenyl, z.B. 2-, 3- oder 4-Nitrophenyl, Cyanphenyl, z.B. 2-, 3- oder 4-Cyanphenyl, Amino- oder Diaminophenyl, z.B. 2-, 3- oder 4-Aminophenyl oder 3,5-Diaminophenyl, Amino-hydroxyphenyl, z.B. 3-Amino-4-hydroxy- oder 4-Amino-2-hydroxyphenyl, Niederalkylaminophenyl, z.B. 2- oder 4-Methylaminophenyl, Diniederalkylaminophenyl, z.B. 2-, 3- oder 4-Dimethylaminophenyl, Niederalkanoylaminophenyl, z.B. 2-, 3- oder 4-Acetylaminophenyl, Sulfamoylphenyl, z.B. 2-, 3- oder 4-Sulfamoylphenyl, Halogenniederalkylphenyl, z.B. 2-, 3- oder 4-Trifluormethylphenyl, oder Aminoniederalkylphenyl, z.B. 2-, 3-oder 4-Aminomethylphenyl, bedeuten, und ihre pharmazeutisch annehmbaren Salze, z.B. die Hydrochloride.

Die Erfindung hat in erster Linie Verbindungen der Formel I zum Gegenstand, worin X die Gruppe $-\overset{\|}{C}-H$, Y Schwefel, A durch veräthertes Hydroxyimino, z.B. Niederalkoxyimino, z.B. Methoxyimino, substituiertes Methylen, $R_1$ Wasserstoff, $R_2$ Wasserstoff oder Niederalkyl, z.B. Methyl, und $R_3$ Furyl, z.B. Fur-2- oder -3-yl, Phenyl oder substituiertes Phenyl, z.B. Niederalkyl-, Diniederalkyl-oder Triniederalkylphenyl, z.B. 2-, 3- oder 4-Methyl-, 2-, 3- oder 4-tert-Butyl-, 3,4-Dimethyl-, 2,3-Dimethyl-, 2,4-Dimethyl- oder 2,4,6-Trimethylphenyl, Hydroxy-, Dihydroxy- oder Trihydroxyphenyl, z.B. 4-Hydroxy-, 2,4-Dihydroxy-, 2,3,4-Trihydroxy-, 2,4,6-Trihydroxy- oder 3,4,5-Trihydroxyphenyl, Hydroxy-niederalkylphenyl, z.B. 2-Hydroxy-3-methylphenyl, Niederalkoxy-, Diniederalkoxy- oder Triniederalkoxyphenyl, z.B. 4-Methoxy-, 3,4-Dimethoxy-, 2,3-Dimethoxy-, 2,4-Dimethoxy- oder 3,4,5-Trimethoxyphenyl, Hydroxy-niederalkoxyphenyl, z.B. 4-Hydroxy-3-methoxyphenyl, Hydroxy-diniederalkoxyphenyl, z.B. 3-Hydroxy-2,4-dimethoxyphenyl, Niederalkanoylphenyl, z.B. 2-, 3- oder 4-Acetylphenyl, Niederalkanoyloxyphenyl, z.B. 2-, 3-, 4- oder 5-Acetoxyphenyl oder 3,4- oder 3,5-Diacetoxyphenyl, Halogenphenyl, z.B. 2-, 3- oder 4-Fluor- oder 2-, 3- oder 4-Chlorphenyl, Nitrophenyl, z.B. 2-, 3-oder 4-Nitrophenyl, Cyanphenyl, z.B. 2-, 3- oder 4-Cyanphenyl, Amino- oder Diaminophenyl,

z.B. 2-, 3- oder 4-Aminophenyl oder 3,5-Diaminophenyl, Aminohydroxyphenyl, z.B. 3-Amino-4-hydroxy- oder 4-Amino-2-hydroxyphenyl, Niederalkylaminophenyl, z.B. 2- oder 4-Methylaminophenyl, Diniederalkylaminophenyl, z.B. 2-, 3- oder 4-Dimethylaminophenyl, Niederalkanoylaminophenyl, z.B. 2-, 3- oder 4-Acetylaminophenyl, Sulfamoylphenyl, z.B. 2-, 3- oder 4-Sulfamoylphenyl, Halogenniederalkylphenyl, z.B. 2-, 3- oder 4-Trifluormethylphenyl, oder Aminoniederalkylphenyl, z.B. 2-, 3- oder 4-Aminomethylphenyl, bedeuten, und ihre pharmazeutisch annehmbaren Salze, z.B. die Hydrochloride.

Die vorliegende Erfindung hat vor allem die in den Beispielen beschriebenen Verbindungen der Formel I und deren pharmazeutisch annehmbaren Salze zum Gegenstand.

Herstellungsverfahren

Verbindungen der Formel I werden beispielsweise hergestellt, indem man

a) in einer Verbindung der Formel:

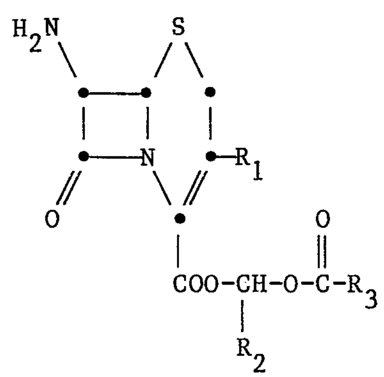

(II),

worin $R_1$, $R_2$ und $R_3$ die unter Formel I genannten Bedeutungen haben, die 7ß-Aminogruppe gegebenenfalls durch eine die Acylierungsreaktion erlaubende Gruppe geschützt ist und eine in $R_2$ oder $R_3$ vorhandene funktionelle Gruppe geschützt ist, die 7ß-Aminogruppe durch Umsetzung mit einem den Acylrest einer Carbonsäure der Formel:

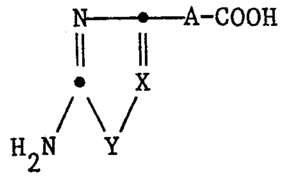

(III)

einführenden Acylierungsmittel, worin A, X und Y und die unter Formel I genannten Bedeutungen haben und die Aminogruppe gegebenenfalls geschützt ist, acyliert, oder

b) eine Verbindung der Formel:

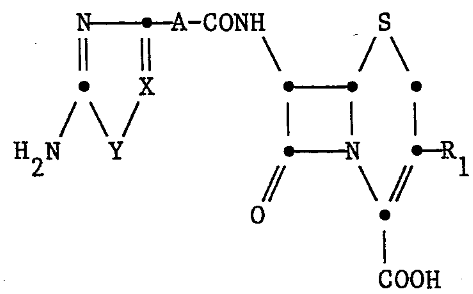

(IV),

worin A, X, Y und $R_1$ die unter Formel I genannten Bedeutungen haben, die Aminogruppe gegebenenfalls und eine in A vorhandene funktionelle Gruppe geschützt ist, oder ein Salz davon oder ein reaktionsfähiges, funktionelles Derivat dieser Verbindung, gegebenenfalls stufenweise, mit einem die Gruppe:

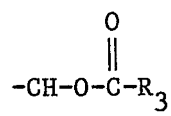

einführenden Veresterungsmittel umsetzt, oder

c) zur Herstellung einer Verbindung der Formel I, worin X, Y, A, $R_2$ und $R_3$ die unter Formel I genannten Bedeutungen haben und $R_1$ Wasserstoff bedeutet, in einer Verbindung der Formel:

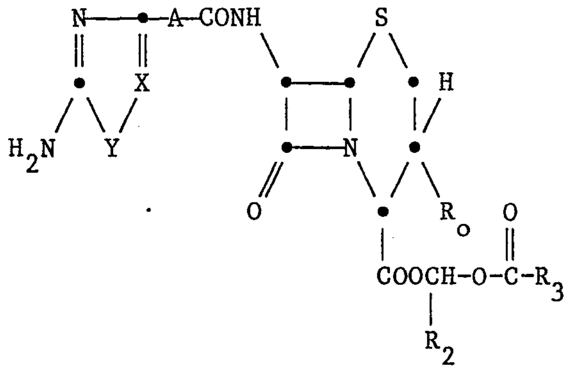

(V),

worin X, Y, A, $R_2$ und $R_3$ die unter Formel I genannten Bedeutungen haben, die Aminogruppe gegebenenfalls und eine in A, $R_2$ oder $R_3$ vorhandene funktionelle Gruppe geschützt ist, $R_o$ eine Abgangsgruppe ist, $R_o$ abspaltet oder

d) ein 1-Oxid der Formel:

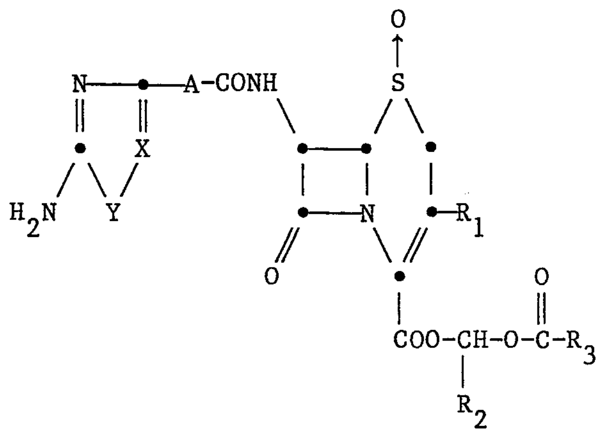

(VI),

worin X, Y, A, $R_1$, $R_2$ und $R_3$ die unter Formel I genannten Bedeutungen haben, die Aminogruppe und eine in A, $R_2$ oder $R_3$ vorhandene funktionelle Gruppe gegebenenfalls geschützt ist, mit einem geeigneten Reduktionsmittel in das 1-Sulfid überführt,

e) zur Herstellung von Verbindungen der Formel I, worin X die Gruppe =C(|)-H und Y Sauerstoff oder Schwefel bedeuten, A, $R_1$, $R_2$ und $R_3$ die unter Formel I genannten Bedeutungen haben, eine Verbindung der Formel:

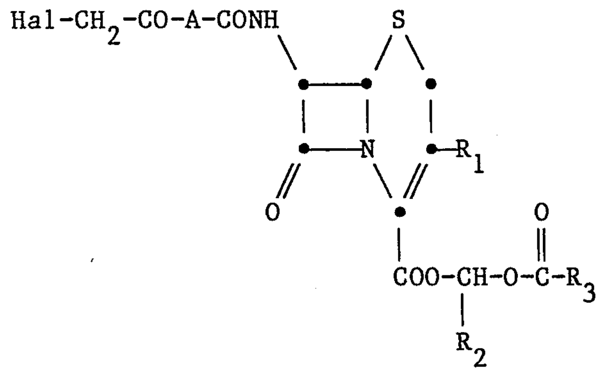

(VII),

worin Hal Halogen bedeutet, A, $R_1$, $R_2$ und $R_3$ die unter Formel I genannten Bedeutungen haben, die Aminogruppe und eine in A, $R_2$ oder $R_3$ vorhandene funktionelle Gruppen gegebenenfalls geschützt ist, mit Harnstoff bzw. Thioharnstoff kondensiert, oder

f) zur Herstellung einer Verbindung der Formel I, worin X Stickstoff und Y Schwefel bedeuten, A, $R_1$, $R_2$ und $R_3$ die unter Formel I genannten Bedeutungen haben, eine Verbindung der Formel:

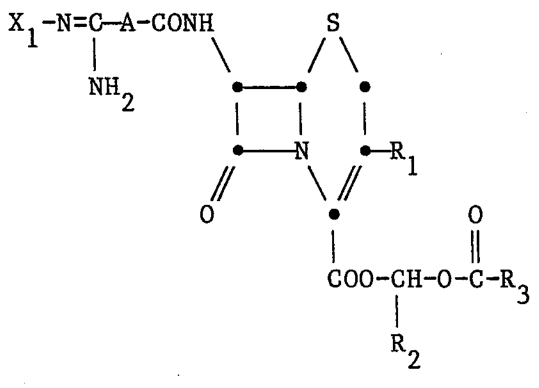

(VIII),

worin $X_1$ Wasserstoff, Halogen oder Hydroxy bedeutet, A, $R_1$, $R_2$ und $R_3$ die unter Formel I genannten Bedeutungen haben, die Aminogruppe und eine in A, $R_2$ oder $R_3$ vorhandene funktionelle Gruppe gegebenenfalls geschützt ist, mit einem Salz der Rhodanwasserstoffsäure oder, falls $X_1$ Wasserstoff ist, mit Dirhodan umsetzt und in einer erhältlichen Verbindung die Aminoschutzgruppe(n) und vorhandene andere Schutzgruppen abspaltet und, wenn erwünscht, eine erhältliche Verbindung in ein Salz oder ein erhältliches Salz in die freie Verbindung oder in ein anderes Salz überführt.

Verfahren a) (Acylierung)

Die 7ß-Aminogruppe in einem Ausgangsmaterial der Formel II ist gegebenenfalls durch eine die Acylierungsreaktion erlaubende Gruppe geschützt. Eine solche Gruppe ist beispielsweise eine organische Silylgruppe, ferner eine Ylidengruppe, die zusammen mit der Aminogruppe eine Schiff'sche Base bildet. Eine organische Silylgruppe ist z.B. eine solche Gruppe, die auch mit einer Carboxygruppe in $R_3$ eine geschützte Carboxygruppe bilden kann, in erster Linie Triniederalkylsilyl, insbesondere Trimethylsilyl. Bei der Silylierungsreaktion zum Schutz der 4-Carboxygruppe in einem Ausgangsmaterial der Formel II kann bei Verwendung eines Ueberschusses des Silylierungsmitels die 7ß-Aminogruppe ebenfalls silyliert werden. Eine Ylidengruppe ist in erster Linie eine 1-Arylniederalkyliden-, insbesondere eine 1-Arylmethylengruppe, worin Aryl besonders für einen carbocyclischen, in erster Linie monocyclischen, Arylrest steht, z.B. für gegebenenfalls durch Niederalkyl, Hydroxy, Niederalkoxy und/oder Nitro substituiertes Phenyl.

In einem Ausgangsmaterial der Formel II ist eine in $R_2$ oder $R_3$ vorhandene funktionelle Gruppe, z.B. eine Amino-, Carboxy- oder Hydroxygruppe, durch eine der weiter vorn genannten Schutzgruppen geschützt.

Ein den Acylrest einer Carbonsäure der Formel III einführendes Acylierungsmittel ist die Carbonsäure der Formel III zusammen mit einem Kondensationsmittel oder ein reaktionsfähiges, funktionelles Derivat oder Salz dieser Carbonsäure.

In einem Ausgangsmaterial der Formel III ist die Aminogruppe am Heterocyclus gegebenenfalls durch eine weiter vorn genannte, übliche Aminoschutzgruppe, z.B. tert-Butoxycarbonyl, geschützt.

In einem Ausgangsmaterial der Formel III kann diese Aminogruppe auch in ionischer Form geschützt sein, z.B. in Form eines Säureadditionssalzes, welches beispielsweise mit einer starken anorganischen

Säure, z.B. einer Halogenwasserstoffsäure, z.B. Salzsäure oder Schwefelsäure, oder mit einer organischen Säure, z.B. p-Toluolsulfonsäure, gebildet wird.

Falls eine freie Säure der Formel III zur Acylierung eingesetzt wird, wird die Reaktion in Gegenwart von geeigneten Kondensationsmitteln, wie Carbodiimiden, beispielsweise N,N'-Diäthyl-, N,N'-dipropyl-, N,N'-dicyclohexyl- oder N-Aethyl-N'-3-dimethylaminopropylcarbodiimid, geeigneten heterocyclischen Carbonylverbindungen, beispielsweise Carbonyldiimidazol, oder 1,2-Oxazoliumverbindungen, wie 2-Aethyl-5-phenyl-1,2-oxazolium-3'-sulfonat oder 2-tert-Butyl-5-methyl-1,2-oxazoliumperchlorat, oder einer geeigneten Acylaminoverbindung, z.B. 2-Aethoxy-1-äthoxycarbonyl-1,2-dihydrochinolin, durchgeführt.

Die Kondensationsreaktion wird vorzugsweise in flüssiger Phase, vorzugsweise in Gegenwart eines Lösungsmitels, z.B. einem aprotischen Lösungsmittel, z.B. Methylenchlorid, Dimethylformamid, Acetonitril oder Tetrahydrofuran, gegebenenfalls unter Kühlen oder Erwärmen, z.B. in einem Temperaturbereich von etwa -40°C bis etwa +100°C, bevorzugt von etwa -20°C bis etwa +50°C, und gegebenenfalls unter Inertgas-, z.B. Stickstoffatmosphäre, durchgeführt.

Ein reaktionsfähiges, funktionelles Derivat einer Carbonsäure der Formel III ist zur Bildung der Carboxamid-Funktion -CONH- befähigt und ist in erster Linie ein Anhydrid, vorzugsweise ein gemischtes Anhydrid der Carbonsäure der Formel III. Ein gemischtes Anhydrid wird beispielsweise durch Kondensation der Carbonsäure der Formel III mit einer anderen Säure, z.B. einer anorganischen Säure, z.B. einer Halogenwasserstoffsäure, gebildet und ist beispielsweise das entsprechende Carbonsäurehalogenid, z.B. das Carbonsäurechlorid oder -bromid. Ein reaktionsfähiges, funktionelles Derivat einer Carbonsäure der Formel III wird ausserdem durch Kondensation mit einer organischen Carbonsäure gebildet, z.B. mit einer unsubstituierten oder durch Halogen, z.B. Fluor oder Chlor, substituierten Niederalkancarbonsäure, z.B. Pivalinsäure oder Trifluoressigsäure, mit einem

Niederalkylhalbester der Kohlensäure, z.B. dem Aethyl- oder Isobutylhalbester der Kohlensäure, oder mit einer organischen, z.B. aliphatischen oder aromatischen, Sulfonsäure, z.B. Methansulfonsäure oder p-Toluolsulfonsäure.

Ein reaktionsfähiges, funktionelles Derivat einer Carbonsäure der Formel III ist ebenfalls ein aktivierter Ester der Carbonsäure der Formel III, der beispielsweise durch Kondensation mit einem substituierten Vinylalkohol, d.h. mit einem Enol, z.B. einem Niederalkenol, gebildet wird, ein Iminomethylesterhalogenid, z.B. Dimethyliminomethylesterchlorid, hergestellt aus der Carbonsäure der Formel III und z.B. Dimethyl-(1-chloräthyliden)-iminiumchlorid der Formel $[(CH_3)_2N^{\oplus}=C(Cl)CH_3]Cl^{\ominus}$, das man seinerseits z.B. aus N,N-Dimethylacetamid und Phosgen oder Oxalylchlorid erhalten kann, ein Arylester, z.B. ein durch Halogen, z.B. Chlor, und/oder Nitro substituierter Phenylester, z.B. der Pentachlor-, 4-Nitrophenyl-oder 2,3-Dinitrophenylester, ein N-heteroaromatischer Ester, z.B. der N-Benztriazolester, oder ein N-Diacyliminoester, z.B. der N-Succinylimino- oder N-Phthalyliminoester.

Die Acylierung mit einem reaktionsfähigen, funktionellen Derivat der Carbonsäure der Formel III, z.B. mit einem entsprechenden Anhydrid, insbesondere einem Säurehalogenid, wird bevorzugt in Gegenwart einer geeigneten Base durchgeführt. Eine geeignete Base ist beispielsweise ein Amin, z.B. ein tertiäres Amin, z.B. Triniederalkylamin, z.B. Trimethylamin, Triäthylamin oder Aethyl-di-isopropylamin, oder ein N,N-Diniederalkylanilin, z.B. N,N-Dimethylanilin, oder ein cyclisches tertiäres Amin, z.B. ein N-Niederalkyl-substituiertes Morpholin, z.B. N-Methylmorpholin, oder ist beispielsweise eine stickstoffhaltige, aromatische Base, z.B. Pyridin. Eine geeignete Base ist ferner eine anorganische Base, beispielsweise ein Alkalimetall-,z.B. Natrium-, -hydroxid, -carbonat- oder -hydrogencarbonat, oder ein Oxiran, beispielsweise ein 1,2-Niederalkylenoxid, wie Aethylenoxid oder Propylenoxid, ferner ein Silylamid, z.B. Trimethylsilylacetamid, ein Carbonsäureamid, z.B. Dimethylformamid, Harnstoff oder ein Nitril, z.B. Acetonitril.

Die Acylierung mit einem reaktionsfähigen, funktionellen Derivat der Carbonsäure der Formel III wird vorzugsweise in einem inerten, vorzugsweise wasserfreien, aprotischen Lösungsmittel oder Lösungsmittelgemisch vorgenommen, beispielsweise in einem Carbonsäureamid, z.B. Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, cyclischen Aether, z.B. Tetrahydrofuran, einem Ester, z.B. Essigsäureäthylester, oder einem Nitril, z.B. Acetonitril, ferner in einem Lösungsmittel, das mit Wasser mischbar ist, z.B. einem Alkohol, z.B. Methanol oder Aethanol, oder Aceton, gegebenenfalls in Anwesenheit von Wasser, gegebenenfalls bei erniedrigter oder erhöhter Temperatur, z.B. in einem Temperaturbereich von etwa -40°C bis etwa +100°C, bevorzugt von etwa -10°C bis etwa +50°C, und gegebenenfalls unter Inertgas-, z.B. Stickstoffatmosphäre.

<u>Verfahren b) (Veresterungen)</u>:
In einem Ausgangsmaterial der Formel IV ist eine in A vorhandene funktionelle Gruppe, z.B. eine Hydroxy- oder Carboxygruppe, durch eine der weiter vorn genannten Schutzgruppen geschützt. Das Salz einer Carbonsäure der Formel IV ist ein Alkalimetall-, z.B. Natrium- oder Kaliumsalz, oder ein Salz mit einer organischen Base, z.B. einem organischen Amin, z.B. einem tertiären Amin, wie Triniederalkylamin, z.B. Trimethylamin, Triäthylamin oder Aethyl-diisopropylamin, ein N,N-Diniederalkylanilin, z.B. N,N-Dimethylanilin, einem cyclischen tertiären Amin, z.B. einem N-niederalkylierten Morpholins, z.B. N-Methylmorpholin, einer Base vom Pyridin-Typ, z.B. Pyridin oder Collidin, oder einem bicyclischen Amidin, z.B. 1,5-Diazabicyclo[5.4.0]undec-5-en (DBU).

Ein die Gruppe:

$$\begin{array}{c} \qquad\quad O \\ \qquad\quad \| \\ -CH-O-C-R_3 \end{array}$$

einführendes Veresterungsmittel ist beispielsweise eine Verbindung der Formel

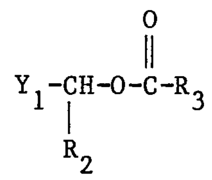

(IX),

worin $Y_1$ eine reaktionsfähige, veresterte Hydroxygrupe ist und $R_2$ und $R_3$ die unter Formel I genannten Bedeutungen haben.

Eine reaktionsfähige, veresterte Hydroxygruppe $Y_1$ in einer Verbindung der Formel IX ist eine durch eine starke anorganische oder organische Säure veresterte Hydroxygruppe. Entsprechende Gruppen $Y_1$ sind insbesondere Halogen, z.B. Chlor, Brom oder bevorzugt Jod, ferner Sulfonyloxygruppen, wie Niederalkan- oder Arylsulfonyloxygruppen, z.B. Methan-, Aethan-, Benzol- oder Toluolsulfonyloxygruppen, oder Halogensulfonyloxygruppen, z.B. die Chlorsulfonyloxygruppe, und dergleichen.

Die Veresterung der 4-Carboxygruppe in einem Ausgangsmaterial der Formel IV wird auf an sich bekannte Weise, z.B. in Gegenwart eines der unter Verfahren a) genannten Kondensationsmittel, z.B. Dicyclohexylcarbodiimid, oder einer Base, z.B. einem bicyclischen Amidin, z.B. Diazabicyclo[5.4.0]undec-5-en, oder einer quaternären Ammoniumbase, z.B. eines Tetraalkylammoniumhydroxids, -carbonats oder -hydrogencarbonats, z.B. worin Alkyl Methyl, Aethyl, Propyl, Isopropyl, Butyl oder dergleichen ist, oder eines Oxirans, beispielsweise eines niederen 1,2-Alkylenoxids, wie Aethylenoxid oder Propylenoxid, durchgeführt.

Eine Verbindung der Formel IV, worin die zu veresternde 4-Carboxygruppe in Form eines reaktionsfähigen, funktionellen Derivates vorliegt, ist beispielsweise ein gemischtes Anhydrid oder ein aktivierter Ester, welcher in der unter Verfahren a) (Acylierung) geschilderten Weise durch Kondensation der Carbonsäure der Formel IV

mit einer anorganischen Säure, einer Carbonsäure, mit einem Halbester der Kohlensäure, einer Sulfonsäure oder durch Kondensation mit einem vinylogen Alkohol erhalten werden kann.

Die Veresterung kann auch stufenweise erfolgen, indem man eine Verbindung der Formel IV mit einem Dihalogenid der Formel:

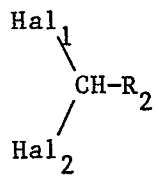

(X),

worin einer der Reste $Hal_1$ und $Hal_2$ Chlor, Brom oder Jod und der andere Brom oder Jod bedeuten, oder ein reaktionsfähiges, funktionelles Derivat einer Verbindung der Formel IV, z.B. das Säurechlorid, mit einem Aldehyd der Formel:

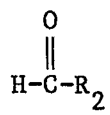

(XI),

oder eine Verbindung der Formel IV mit einem reaktionsfähigen Derivat des Aldehyds der Formel XI, z.B. einem Acetal, umsetzt und die erhaltene Verbindung der Formel:

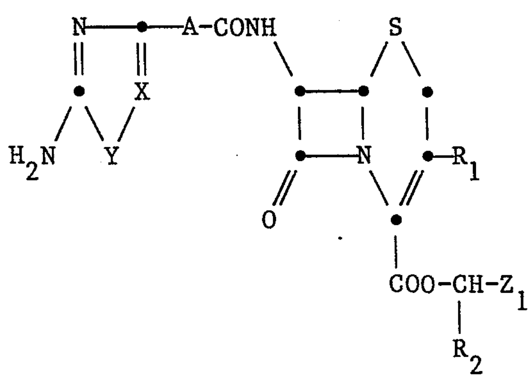

(XII),

worin $Z_1$ Hydroxy oder eine reaktionsfähige, veresterte Hydroxygruppe ist, in situ oder nach Isolierung mit einer Carbonsäure der Formel:

$$HO-\overset{O}{\overset{\|}{C}}-R_3 \qquad (XIII),$$

oder einem Salz dieser Carbonsäure, z.B. einem Alkalimetall-, z.B. dem Natriumsalz·oder, wenn $Z_1$ Hydroxy ist, mit einem reaktionsfähigen, funktionellen Derivat dieser Carbonsäure, z.B. dem Säurechlorid, umsetzt.

Die Umsetzung einer Verbindung der Formel IV mit dem Dihalogenid der Formel X erfolgt in Gegenwart einer der unter Verfahren a) genannten Basen oder Kondensationsmittel.

Die Umsetzung des reaktionsfähigen, funktionellen Derivats der Verbindung der Formel IV mit einem Aldehyd der Formel XI kann in Gegenwart von Zinkchlorid nach der von Ulich C.H. und Adams R. in JACS 43, S. 660 (1921) angegebenen Methode erfolgen.

Die Verbindungen der Formel XII und ihre pharmazeutisch annehmbaren, nicht-toxischen Salze sind wertvolle, antibiotisch wirksame Stoffe, die insbesondere als antibakterielle Antibiotika verwendet werden können. So sind Verbindungen der Formel XII in vivo bei peroraler Applikation an der Maus gegen systemische Infektionen durch grampositive Keime, z.B. Streptococcus pyogenes, oder gramnegative Keime, z.B. Enterobakterien, wirksam. Die Verbindungen der Formel XII können daher als orale, antibakterielle Antibiotika, z.B. in Form von oral verabreichbaren, pharmazeutischen Präparaten, z.B. Kapseln, Tabletten, Sirupen oder dergleichen, zur Bekämpfung von Infektionen verwendet werden. Ausserdem zeichnen sich die Verbindungen der Formel XII durch eine überraschend gute Resorption nach oraler Verabreichung aus, was durch hohe renale Ausscheidung nachgewiesen werden kann.

Die Umsetzung einer Verbindung der Formel XII, worin $Z_1$ Hydroxy ist, mit einem reaktionsfähigen, funktionellen Derivat einer Carbonsäure der Formel XIII, oder einer Verbindung der Formel XII, worin $Z_1$ eine

reaktionsfähige, veresterte Hydroxygruppe ist, mit einer Carbonsäure der Formel XIII oder einem Salz dieser Carbonsäure erfolgt mit den unter Verfahren a) genannten Kondensationsmitteln oder Basen.

Die Veresterungsreaktion wird in einem geeigneten Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril, oder in einem Gemisch von nicht mischbaren Lösungsmitteln, z.B. Methylenchlorid/Wasser oder Diäthyläther/Wasser (Phasentransfer). Die Umsetzungen werden bei Raumtemperatur, wenn notwendig bei erniedrigter oder erhöhter Temperatur, vorgenommen, etwa bei -40°C bis zur Siedetemperatur des Reaktionsgemisches, bevorzugt bei -10°C bis +40°C, und, wenn notwendig, in einer Inertgas-, z.B. Stickstoffatmosphäre.

<u>Verfahren c) ($R_o$-Abspaltung):</u>

In einem Ausgangsmaterial der Formel V ist eine in A, $R_1$ oder $R_3$ vorhandene funktionelle Gruppe, z.B. eine Amino-, Hydroxy- oder Carboxygruppe, durch eine der weiter vorn genannten Schutzgruppen geschützt. Die Abgangsgruppe $R_o$ ist Hydroxy oder Amino und bevorzugt verestertes Hydroxy oder substituiertes Amino. Eine veresterte Hydroxygruppe $R_o$ kann durch eine anorganische oder organische Säure, wie eine starke Mineralsäure, z.B. eine Halogenwasserstoffsäure, wie Chlorwasserstoff-, Bromwasserstoff-oder Jodwasserstoffsäure, oder eine organische Carbon- oder Sulfonsäure, inklusive Ameisensäure, z.B. eine aliphatische, cycloaliphatische, cycloaliphatisch-aliphatische, aromatische, araliphatische, heterocyclische oder heterocyclisch-aliphatische Säure, ferner durch ein Kohlensäurehalbderivat, verestert sein. So stellt $R_o$ z.B. Halogen, wie Chlor, Brom oder Jod, Niederalkylsulfonyloxy, z.B. Methylsulfonyloxy oder Aethylsulfonyloxy, Arylsulfonyloxy, z.B. Benzolsulfonyloxy, oder p-Toluolsulfonyloxy, Niederalkanoyloxy, z.B. Acetoxy oder Propionyloxy, Arylcarbonyloxy, z.B. Benzoyloxy, oder Niederalkoxycarbonyloxy, z.B. Methoxycarbonyloxy oder Aethoxycarbonyloxy, dar. Eine substituierte Aminogruppe $R_o$ ist eine sekundäre oder tertiäre Aminogruppe $-N(R_o^1)(R_o^2)$, worin einer der Reste $R_o^1$ und $R_o^2$ Wasserstoff und der andere Rest oder auch beide Reste $R_o^1$ und $R_o^2$ einen gegebenenfalls substituierten aliphatischen, cycloaliphatisch-aliphatischen, aromatischen, araliphatischen, heterocyclischen oder heterocyclisch-aliphatischen Rest, oder wenn $R_o^1$ und $R_o^2$ verbunden sind, eine gegebenenfalls substituierte, gegebenenfalls durch Heteroatome, wie Sauerstoff, Schwefel oder Stickstoff, unterbrochene Alkylengruppe bedeuten. Solche substituierten sekundären oder tertiären Aminogruppen sind beispielsweise Mono- oder Diniederalkylamino, wie Methyl-, Aethyl-, Propyl-, Dimethyl-, Diäthyl-, Dipropyl-, Methyläthyl- oder Methylpropylamino, Mono-oder Dicyclohexylamino, Diphenylamino, Benzyl-, Dibenzyl- oder Phenyläthyl- oder Diphenyläthylamino, Aziridino, Pyrrolidino, Piperidino, Morpholino, Thiamorpholino oder 4-Niederalkylpiperazino.

Die Abspaltung einer Verbindung der Formel $R_o$-H, d.h. von Wasser, einer Säure oder eines Amins, wird vorzugsweise in Gegenwart einer Säure, z.B. einer starken organischen Carbon- oder Sulfonsäure, wie einer Halogenniederalkancarbonsäure, z.B. Trifluoressigsäure, oder einer Arylsulfonsäure, z.B. p-Toluolsulfonsäure, eines reaktionsfähigen Säurederivats, z.B. eines Anhydrids oder insbesondere eines Säurehalogenids, z.B. Säurechlorids, einer anorganischen, z.B. Phosphor-Sauerstoff- oder Schwefel-Sauerstoffsäure, z.B. Phosporoxychlorid oder Thionylchlorid, durchgeführt. Bei der Abspaltung von Wasser verwendet man eine Base, z.B. eine tertiäre organische Base, z.B. Pyridin, oder einen geeigneten sauren Ionenaustauscher, z.B. auf Sulfonsäure-Basis, z.B. einen sulfonierten Polystyrolionenaustauscher. Zur Abspaltung von Wasser kann man auch Kondensationsmittel wie Carbodiimidverbindungen, z.B. Dicyclohexylcarbodiimid, verwenden. Die Abspaltung erfolgt in Gegenwart eines Lösungsmittels, wie eines gegebenenfalls halogenierten, aliphatischen, cycloaliphatischen oder aromatischen Kohlenwasserstoffs, z.B. Benzol oder

Toluol, oder eines Lösunsmittelgemisches, wobei man flüssige Säuren, wie Trifluoressigsäure, gleichzeitig auch als Lösungsmittel verwenden kann. Wenn notwendig, verwendet man zusätzlich ein Wasser-absorbierendes Mittel oder einen Wasserabscheider und arbeitet unter Kühlen oder Erwärmen und/oder in einer Inertgas-, z.B. Stickstoffatmosphäre.

Vorzugsweise bedeutet $R_o$ in einem Ausgangsmaterial der Formel V eine veresterte Hydroxygruppe. Ueblicherweise verwendet man für die Abspaltung dieser Gruppe Protonenakzeptoren, z.B. Basen, z.B. anorganische Basen, z.B. verdünnte wässrige Alkalimetallhydroxide, z.B. Natrium- oder Kaliumhyroxid, wobei man neben Wasser auch organische Lösungsmittel, wie geeignete Ketone, z.B. Aceton, oder Aether, z.B. Dioxan, oder wässrige Gemische davon verwenden und bei einem pH-Wert von höchstens etwa 9, wenn notwendig unter Kühlen oder Erwärmen und/oder in einer Inertgas-, z.B. Stickstoffatmosphäre, arbeiten kann.

Vorzugsweise verwendet man als Protonenakzeptoren tert-Amine, insbesondere Protonenakzeptoren, die den Lactamring nicht angreifen, in erster Linie tert-aliphatische oder tert-cycloaliphatische Mono-und Diamine, wie Triniederalkylamine, z.B. Trimethylamin, Triäthylamin oder Aethyl-diisopropylamin oder bicyclische Amidine, z.B. 1,5-Diazabicyclo[4,3,0]non-5-en oder 1,5-Diazabicyclo[5,4,0]-undec-5-en. Ferner kann man basische Ionenaustauscher, z.B. auf Ammoniumhydroxid-Basis, als Protonenakzeptoren einsetzen. Gewisse veresterte Hydroxygruppen $R_o$, insbesondere Sulfonyloxy, z.B. die Methansulfonyloxygruppe, lassen sich in Form einer Säure der Formel $R_o$-H auch durch Absorption, z.B. an Silikagel, Aluminiumoxyd etc., und Elution (Chromatographie), aus Verbindungen der Formel V abspalten.

Verfahren d) (Reduktion des 1-Oxids):

In einem Ausgangsmaterial der Formel VI ist eine in A, $R_1$ oder $R_3$ vorhandene funktionelle Gruppe, z.B. eine Amino-, Hydroxy- oder Carboxygruppe gegebenenfalls durch eine der weiter vorn genannten Schutzgruppen geschützt.

Die Reduktion des 1-Oxids der 3-Cephem-Verbindung der Formel VI kann in an sich bekannter Weise durch Behandeln mit einem geeigneten Reduktionsmittel, wenn notwendig in Anwesenheit eines aktivierenden Mittels, durchgeführt werden. Geeignete Reduktionsmittel sind beispielsweise: Reduzierend wirkende Metallkationen, z.B. Zinn-, Eisen-, Kupfer- oder Mangankationen, welche in Form ihrer Salze, z.B. als Zinn-II-chlorid, -acetat oder -formiat, Eisen-II-chlorid, -sulfat, -oxalat, oder Ammoniumeisensulfat, Kupfer-I-chlorid oder -oxid, oder Mangan-II-chlorid, -sulfat, -acetat oder -oxid, oder als organische oder anorganische Komplexe, z.B. mit Aethylendiamintetraessigsäure oder Nitrilotriessigsäure, verwendet werden; reduzierend wirkende Dithionit-, Jod- oder Eisen-II-cyanidsalze, z.B. Alkalimetall-, z.B. Natrium- oder Kaliumdithionit, Natrium- oder Kaliumjodid oder Kaliumeisen-II-cyanid; Jodwasserstoffsäure, reduzierend wirkende trivalente, anorganische oder organische Phosporverbindungen, wie Phosphine, ferner Ester, Amide und Halogenide der phosphonigen, phosphinigen oder phosporigen Säure, sowie diesen Phosphor-Sauerstoffverbindungen entsprechende Phospor-Schwefelverbindungen, worin in diesen Verbindungen organische Reste in erster Linie aliphatische, aromatische oder araliphatische Reste, z.B. gegebenenfalls substituiertes Niederalkyl, Phenyl oder Phenylniederalkyl, darstellen, z.B. Triphenylphosphin, Diphenylphosphonigsäuremethylester, Diphenylchlorphosphin, Phenyldichlorphosphin,, Benzolphosphonigsäuredimethylester, Phosphorigsäuretriphenylester, Phosphorigsäuretrimethylester, Phosphortrichlorid, Phosphortribromid, ferner Phosphorigsäuretriphenylester-Halogenaddukte, z.B. Chlor- oder Bromaddukte, worin die Phenylreste gegebenenfalls durch Niederalkyl, z.B. Methyl, Niederalkoxy, z.B. Methoxy, oder durch Halogen, z.B. Chlor, substituiert sind, etc.; reduzierend wirkende Halogensilanverbindungen, die mindestens ein an das Siliciumatom gebundendes

Wasserstoffatom aufweisen, und die ausser Halogen, wie Chlor, Brom oder Jod, auch organische Reste, wie aliphatische oder aromatische Gruppen, z.B. gegebenenfalls substituiertes Niederalkyl oder Phenyl, aufweisen können, beispielsweise Diphenylchlorsilan oder Dimethylchlorsilan, sowie auch Halogensilanverbindungen, worin alle Wasserstoffe durch organische Reste ersetzt sind, wie Triniederalkylhalogensilan, z.B. Trimethylchlorsilan oder Trimethyljodsilan, etc.; reduzierend wirkende quaternäre Chlormethyleniminiumsalze, insbesondere -chloride oder -bromide, worin die Iminiumgruppe durch einen bivalenten oder zwei monovalente organische Reste, wie gegebenenfalls substituiertes Niederalkylen bzw. Niederalkyl substituiert ist, wie N-Chlormethylen-N,N-dimethyliminiumchlorid oder N-Chlormethylenpyrrolidiniumchlorid; oder komplexe Metallhydride, wie Natriumborhydrid, in Gegenwart von geeigneten Aktivierungsmitteln, wie Kobalt-II-chlorid.

Aktivierende Mittel verwendet man zusammen mit solchen Reduktionsmitteln, welche keine oder nur schwache Lewissäure-Eigenschaften aufweisen. Diese werden in erster Linie zusammen mit Dithionit-, Jod- oder Eisen-II-cyanidsalzen und nicht halogenhaltigen trivalenten Phosphorreduktionsmitteln eingesetzt und sind insbesondere organische Carbon- und Sulfonsäurehalogenide, z.B. Phosgen, Oxalylchlorid, Essigsäurechlorid oder -bromid, oder Chloressigsäurechlorid.

Die Reduktion wird vorzugsweise in Gegenwart von Lösungsmitteln oder Gemischen davon durchgeführt, deren Auswahl in erster Linie durch die Löslichkeit der Ausgangsstoffe und die Wahl des Reduktionsmittels bestimmt wird, z.B. gegebenenfalls substituierte, z.B. halogenierte oder nitrierte, aliphatische, cycloaliphatische, aromatische oder araliphatische Kohlenwasserstoffe, z.B. Benzol, Methylenchlorid, Chloroform oder Nitromethan, geeignete Säurederivate, wie Niederalkancarbonsäureester oder -nitrile, z.B. Essigsäureäthylester oder Acetonitril, oder Amide von anorganischen oder organischen Säuren, z.B. Dimethylformamid oder Hexamethylphosphoramid, Aether, z.B. Diäthyläther, Tetrahydrofuran oder Dioxan, Ketone, z.B. Aceton,

oder Sulfone, insbesondere aliphatische Sulfone, z.B. Dimethylsulfon oder Tetramethylensulfon, etc., zusammen mit den chemischen Reduktionsmitteln, wobei diese Lösungsmittel vorzugsweise kein Wasser enthalten.

Dabei arbeitet man gewöhnlicherweise bei Temperaturen von etwa -20°C bis etwa 100°C, wobei man bei Verwendung von sehr reaktionsfähigen Reduktiosmiteln bzw. Aktivierungsmitteln die Reaktion auch bei tieferen Temperaturen durchführt und gegebenenfalls unter Inertgasatmosphäre, z.B. Stickstoffatmosphäre.

Die oben beschriebenen Säureabspaltungen können, falls die Reaktionspartner flüssig sind, ohne Lösungsmittel, üblicherweise aber in Gegenwart eines Lösungsmittels, wie eines gegebenenfalls halogenierten Kohlenwasserstoffs, z.B. eines aliphatischen, cycloaliphatischen oder aromatischen Kohlenwasserstoffs, wie Methylenchlorid, Cyclohexan oder Toluol, eines Niederalkanols, z.B. Aceton, oder Aethers, z.B. Tetrahydrofuran oder Dioxan, oder eines Lösungsmittelgemisches, inkl. eines wässrigen Gemisches, wenn notwendig unter Kühlen oder Erwärmen, bei -30°C bis 150°C. bevorzugt bei etwa -10°C bis 60°C, und/oder in einer Inertgas-, z.B. Stickstoffatmosphäre, durchgeführt werden.

<u>Verfahren e) (Ringschluss)</u>:
In einem Ausgangsmaterial der Formel VII ist eine in A, $R_1$, $R_2$ und $R_3$ vorhandene funktionelle Gruppe, z.B. eine Amino-, Hydroxy- oder Carboxygruppe, gegebenenfalls durch eine der weiter vorn genannten Schutzgruppen geschützt. Hal bedeutet Halogen, bevorzugt Chlor, ferner Brom, Jod oder Fluor. Wenn im Produkt der Formel I Y Sauerstoff bedeuten soll, wird Harnstoff, wenn Y Schwefel bedeutet, Thioharnstoff, in äquivalenter Menge oder im Ueberschuss eingesetzt.

Die Zyklisierung wird im allgemeinen in einem Lösungsmittel, wie Wasser oder einem organischen, inerten lösungsmittel oder einer Mischung davon, durchgeführt. Als organische Lösungsmittel geeignet sind Alkohole, wie Methanol, Aethanol oder Isopropanol, Ketone, wie

Aceton, Aether, wie Dioxan oder Tetrahydrofuran, Nitrile, wie Acetonitril, halogenierte Kohlenwasserstoffe, wie Methylenchlorid, Chloroform oder Tetrachlorkohlenstoff, Ester, wie Aethylacetat, oder Amide, wie Dimethylformamid oder Dimethylacetamid, und ähnliche. Die Reaktion kann in Gegenwart einer unter Verfahren a) genannten Base durchgeführt werden. Die Reaktionstemperatur liegt zwischen Raumtemperatur und der Siedetemperatur des Reaktionsgemisches, vorzugsweise zwischen 60°C und der Siedetemperatur des Reaktionsgemisches.

Die Zyklisierung kann auch stufenweise erfolgen, indem zunächst ein offenes Zwischenprodukt, z.B. der Teilformel $NH_2$-C(=NH)-O-$CH_2$-CO-A- bzw. $NH_2$-C(=NH)-S-$CH_2$-CO-A- entsteht, das dann in der zweiten Stufe durch Erwärmen dehydratisiert wird.

Verfahren f) (Ringschluss):

In einem Ausgangsmaterial der Formel VIII ist eine in A, $R_1$ ,$R_2$ und $R_3$ vorhandene funktionelle Gruppe, z.B. eine Amino-, Hydroxy- oder Carboxygruppe, gegebenenfalls durch eine der weiter vorn genannten Schutzgruppen geschützt. $X_1$ mit der Bedeutung Halogen ist bevorzugt Brom, ferner Fluor, Chlor oder Jod. Geeignete Salze der Rhodanwasserstoffsäure sind beispielsweise Alkali-, Erdalkali- oder Schwermetallrhodanide, z.B. das Natrium-, Kalium-, Magnesium- oder Bleirhodanid, oder auch Ammoniumrhodanide, worin die Ammoniumgruppe von Ammoniak oder von organischen Stickstoffbasen abgeleitet ist, z.B. Ammonium- oder Mono-, Di-, Tri- oder Tetraniederalkylammoniumrhodanid, worin Niederalkyl z.B. Methyl, Aethyl oder Isopropyl bedeutet.

Die Reaktion wird bevorzugt in einem inerten Lösungsmittel, wie einem Niederalkanol, z.B. Methanol, Aetanol, Propanol oder Isopropanol, einem Aether, wie Diäthyläther, Dioxan oder Tetrahydrofuran, einem Amid, wie Dimethylformamid, einem aliphatischen oder aromatischen Kohlenwasserstoff, wie Hexan, Benzol oder Toluol, oder auch in Mischungen davon, und, wenn notwendig, unter Kühlen oder Erwärmen, d.h. bei Temperaturen zwischen etwa -8°C bis zur Siedetemperatur des

Reaktionsgemisches, bevorzugt zwischen etwa +20°C, und Siedetemperatur des Reaktionsgemisches, und/oder in einer Inertgasatmosphäre durchgeführt.

Das Ausgangsmaterial der Formel VIII, worin $X_1$ Halogen ist, kann in situ hergestellt werden, gegebenenfalls in Gegenwart des Salzes der Rhodanwasserstoffsäure, beispielsweise indem man eine Verbindung der Formel VIII, worin $X_1$ Wasserstoff ist, in einem der genannten Lösungsmittel mit einem Hypohalogenit, z.B. Natrium- oder Kaliumhypobromit, oder mit einem Halogen und einer Alkalimetallbase, wie einem Hydroxid, Carbonat oder Niederalkanolat, z.B. mit Brom und Natriummethylat oder Kaliumcarbonat, behandelt. Die Halogenierung findet bevorzugt unter Kühlen, z.B. bei einer Temperatur von etwa -15°C bis etwa 0°C statt.

Das Ausgangsmaterial der Formel VIII, worin X Hydroxy ist, kann ebenfalls in situ hergestellt werden, beispielsweise indem man eine Verbindung der Formel VIII, worin X Wasserstoff ist, mit einem Oxydationsmittel, wie Wasserstoffperoxid, behandelt.

Die Reaktion des Ausgangsmaterials der Formel VIII, worin X Wasserstoff ist, mit Dirhodan wird vorzugsweise ebenfalls in einem der genannten inerten Lösungsmittel und unter ähnlichen Reaktionsbedingungen durchgeführt. Gegebenenfalls kann auch ein Säureadditionssalze einer Verbindung der Formel VIII eingesetzt werden, das in situ in die freie Verbindung übergeführt wird, oder das Dirhodan kann in situ gebildet werden, z.B. aus einem Rhodanid, z.B. Bleirhodanid, und einem Halogen, z.B. Brom.

Nachoperationen

Substituierung der Hydroxyiminogruppe:

In einer erhältlichen Verbindung der Formel I, worin X, Y, $R_1$, $R_2$ und $R_3$ die unter Formel I genannten Bedeutungen haben und A eine durch Hydroxyimino substituierte Methylengruppe bedeutet, kann man die Hydroxyiminogruppe substituieren und so zu Verbindungen der

Formel I gelangen, worin A durch veräthertes oder verestertes Hydroxyimino substituiertes Methylen bedeutet. Alle funktionellen Gruppen ausser der Hydroxyiminogruppe sind geschützt.

Zur Substitution der Hydroxyiminogruppe eignen sich Diazoniederalkane, z.B. Diazomethan, veresterte Niederalkanole oder Cycloalkanole, z.B. Ester mit starken anorganischen Säuren, z.B. Niederalkyl-, z.B. Methyl-, oder Cycloalkyl-, z.B. Cyclopropyl-, -halogenide, z.B. -chloride, -bromide oder -iodide, Diniederalkylsulfate, z.B. Dimethylsulfat, Fluorsulfonsäureniederalkylester, z.B. -methylester, Halogensubstituierte Methansulfonsäureniederalkylester, z.B. Trifluormethansulfonsäuremethylester, geeignete Acetale, z.B. geminale Diniederalkoxyniederalkane, z.B. 2,2-Dimethoxypropan, Triniederalkyloxoniumsalze (sogenannte Meerwein-Salze), z.B. Trimethyloxoniumhexafluorantimonat, Diniederalkoxycarbeniumsalze, z.B. Dimethoxycarbeniumhexafluorphosphat, Diniederalkylhaloniumsalze, z.B. Dimethylbromoniumhexafluoratimonat, 1-Niederalkyl-3-aryltriazen-Verbindungen, z.B. 1-Methyl-3-(4-methylphenyl)-triazen. diese Reagenzien werden in an sich bekannter Weise als Alkylierungsmittel verwendet, üblicherweise in einem geeigneten, wasserfreien Lösungsmittel, z.B. in Benzol, Tetrachlorkohlenstoff oder Tetrahydrofuran, wenn notwendig in Gegenwart eines Kondensationsmittels, z.B. einer Base, z.B. 1,5-Diazabicyclo[5.4.0]undec-5-en, unter Kühlen bis auf -50°C oder Erwärmen bis zur Siedetemperatur des Reaktionsgemisches und gegebenenfalls unter Schutzgasatmosphäre, z.B. Stickstoffatmosphäre.

Die Veresterung der Hydroxyiminogruppe erfolgt auf an sich bekannte Weise, z.B. durch Carbamoylierung. Dabei wird eine Verbindung der Formel I, worin A durch Hydroxyimino substituiertes Methylen bedeuten, und worin funktionelle Gruppen in geschützter Form vorliegen, mit einem Carbamoylierungsmittel behandelt. Geeignete Carbamoylierungsmittel sind reaktionsfähige Derivate von Carbaminsäuren, insbesondere Isocyanate oder gemischte Anhydride, z.B. Carbaminsäurechloride, die man in situ aus Phosgen und einem Amin herstellen kann.

Die Umsetzung mit Isocyanaten erfolgt in einem wasserfreien, inerten Lösungsmittel, beispielsweise einem gegebenenfalls halogenierten Kohlenwasserstoff, z.B. Benzol, Xylol oder Methylenchlorid, einem Aether, z.B. Diäthyläther, Dioxan oder Tetrahydrofuran, oder einem Ester, z.B. Essigsäureäthylester, gegebenenfalls in Gegenwart eines basischen Katalysators, beispielsweise eines tertiären Amins, z.B. Triäthylamin oder Pyridin, bei Raumtemperatur oder etwas erniedrigter oder erhöhter Temperatur, etwa zwischen -20°C und +50°C.

Zum Umsatz mit einem gemischten Anhydrid der Carbaminsäure wird die Hydroxyiminogruppe vorzugsweise vorher metallisiert, beispielsweise mit einem Alkalimetallhydrid, z.B. Natrium- oder Lithiumhydrid. Die an der Hydroxygruppe metallisierte Verbindung der Formel I kann auch zunächst mit Phosgen und anschliessend mit dem Amin $R-NH_2$ behandelt werden und auf diese Art stufenweise carbamoyliert werden. Diese Reaktionen finden ebenfalls in einem inerten Lösungsmittel und bei den oben angegebenen Temperaturen statt.

Abspaltung von Schutzgruppen

In einer erhältlichen Verbindung der Formel I, worin eine oder mehrere funktionelle Gruppen geschützt sind, können diese Schutzgruppen, z.B. eine geschützte Carboxy-, Amino- oder Hydroxygruppe, in an sich bekannter Weise, z.B. duch Solvolyse, insbesondere Hydrolyse, Alkoholyse oder Acidolyse, oder durch Reduktion, insbesondere Hydrogenolyse, gleichzeitig oder selektiv freigesetzt werden.

Eine geschützte Carboxygruppe setzt man in an sich bekannter und je nach Art der Schutzgruppen in verschiedenartiger Weise, vorzugsweise mittels Solvolyse oder Reduktion, frei.

So kann man tert-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln

mit einer geeigneten Säure, wie Ameisensäure oder Trifluoressigsäure, gegebenenfalls unter Zugabe einer nukleophilen Verbindung, wie Phenol, Anisol oder Aethylenthioglykol, in freies Carboxy überführen. Geeignet substituiertes Benzyloxycarbonyl, wie 4-Nitrobenzyloxycarbonyl, kann man durch chemische Reduktion in freies Carboxy überführen, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoffionen-abgebenden Mittels, das zusammen mit dem Metall oder Metallsalz nascierenden Wasserstoff zu erzeugen vermag, wie einer Säure, in erster Linie einer geeigneten Carbonsäure, wie einer gegebenenfalls, z.B. durch Hydroxy substituierten Niederalkancarbonsäure, z.B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt. Durch Behandeln mit einem reduzierenden Metall oder Metallsalz kann man, wie oben beschrieben, auch 2-Halogenniederalkoxycarbonyl, gegebenenfalls nach Umwandlung einer 2-Bromniederalkoxycarbonylgruppe in die entsprechende 2-Jodniederalkoxycarbonylgruppe, oder Aroylmethoxycarbonyl in freies Carboxy umwandeln, wobei Aroylmethoxycarbonyl ebenfalls durch Behandeln mit einem nucleophilen, vorzugsweise salzbildenden, Reagenz, wie Natriumthiophenolat oder Natriumjodid, gespalten werden kann. Substituiertes 2-Silyläthoxycarbonyl kann auch durch Behandeln mit einem, das Fluoridanion liefernden Salz der Fluorwasserstoffsäure, wie einem Alkalimetallfluorid, z.B. Natrium-oder Kaliumfluorid, in Anwesenheit eines macrocyclischen Polyäthers ("Kronenäther"), oder mit einem Fluorid einer organischen quaternären Base, wie Tetraniederalkylammoniumfluorid oder Triniederalkylarylammoniumfluorid, z.B. Tetraäthylammoniumfluorid oder Tetrabutylammoniumfluorid, in Gegenwart eines aprotischen polaren Lösungsmittels, wie Dimethylsulfoxid oder N,N-Dimethylacetamid, in freies Carboxy übergeführt werden. Mit einer organischen Silylgruppe, wie Triniederalkylsilyl, z.B. Trimethylsilyl, verestertes Carboxy, kann üblicherweise solvolytisch, z.B. durch Behandeln mit Wasser, einem Alkohol oder einer Säure, freigesetzt werden.

Eine geschützte Aminogruppe setzt man in an sich bekannter und je nach Art der Schutzgruppen verschiedenartiger Weise, vorzugsweise mittels Solvolyse oder Reduktion, frei. 2-Halogenniederalkoxycarbonylamino kann, gegebenenfalls nach Umwandlung einer 2-Bromniederalkoxycarbonylaminogruppe in die 2-Jodniederalkoxycarbonylaminogruppe, Aroylmethoxycarbonylamino oder 4-Nitrobenzyloxycarbonylamino durch Behandeln mit einem geeigneten Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z.B. Natriumdithionit, gespalten werden. Gegebenenfalls substituiertes Diphenylmethoxycarboylamino, tert-Niederalkoxycarbonylamino, z.B. tert-Butoxycarbonylamino (BOC), 2-trisubstituiertes Silyläthoxycarbonylamino oder 4-Nitrophenylthio kann durch Behandeln mit einer geeigneten Säure, z.B. Ameisen- oder Trifluoressigsäure, und eine mit einer organischen Silylgruppe geschützte Aminogruppe, z.B. mittels Hydrolyse oder Alkoholyse, gespalten werden. Eine durch 2-Halogenacetyl, z.B. 2-Chloracetyl, geschützte Aminogruppe kann durch Behandeln mit Thioharnstoff in Gegenwart einer Base und anschliessende Solvolyse, wie Alkoholyse oder Hydrolyse, des entstandenen Kondensationsproduktes gespalten werden. Eine durch 2-substituiertes Silyläthoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe anggeben, in die freie Aminogruppe übergeführt werden.

In Form einer Azidogruppe geschütztes Amino wird z.B. durch Reduktion in freies Amino übergeführt, z.B. durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser

oder einem Gemisch von Wasser und einem organischen Lösungsmittel, wie einem Alkohol oder Dioxan, bei etwa 20°C bis 25 °C, oder auch unter Kühlen oder Erwärmen, durchgeführt.

Die beschriebenen Spaltungsreaktionen werden im übrigen unter an sich bekannten Bedingungen durchgeführt, wenn notwendig unter Kühlen oder Erwärmen und, wenn notwendig, in einer Inertgas-. z.B. Stickstoffatmosphäre.

Bevorzugt werden selektive Abspaltungsreaktionen durchgeführt, d.h. solche, bei denen die Estergruppierung und die Methoxyiminogruppe entweder gar nicht oder nur wenig angegriffen werden. Eine solche bevorzugte Methode ist die acidolytische Abspaltung einer tert-Niederalkoxycarbonylgruppe, insbesondere der tert-Butoxycarbonylgruppe, mittels einer geeigneten Säure, insbesondere Trifluoressigsäure, gegebenenfalls in einem inerten Lösungsmittel, wie einem gegebenenfalls halogenierten Kohlenwasserstoff, z.B. Methylenchlorid, bei Temperaturen von etwa -20°C bis etwa 50°C, bevorzugt bei 0°C bis Raumtemperatur.

Salzbildung:

Salze von Verbindungen der Formel I können in an sich bekannter Weise hergestellt werden. So kann man Salze von Verbindungen der Formel I z.B. durch Reaktion der sauren Gruppen mit Metallverbindungen, wie Alkalimetallsalzen von geeigneten Carbonsäuren, z.B. dem Natriumsalz der α-Aethylcapronsäure, mit einem Alkalimetall-, z.B. Natriumcarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einem oder mehreren Aequivalenten einer Säure, z.B. Milchsäure, Zitronensäure oder Weinsäure oder einem geeigneten Anionenaustauschreagenz. Innere Salze von Verbindungen der Formel I können z.B. durch Neutralisieren

von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Gemische von Isomeren können in an sich bekannter Weise, z.B. durch fraktionierte Kristallisation, Chromatographie etc., in die einzelnen Isomere aufgetrennt werden.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den vorstehend als besonders bevorzugt aufgeführten Verbindungen gelangt.

Pharmazeutische Präparate:

Die pharmakologisch verwendbaren Verbindungen der Formel I, deren Hydrate oder Salze können zur Herstellung von pharmazeutischen Präparaten verwendet werden.

Pharmazeutische Präparate enthalten eine wirksame Menge des reinen Wirkstoffes der Formel I selbst oder eine wirksame Menge des Wirkstoffes der Formel I im Gemisch mit anorganischen oder organischen, festen oder flüssigen, pharmazeutisch verwendbaren Trägerstoffen, die sich zur peroralen Verabreichung eignen.

Zur peroralen Verabreichung verwendet man Tabletten oder Gelatinekapseln, welche den Wirkstoff zusammen mit Zusatzstoffen, z.B. Laktose, Dextrose, Sukrose, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, enthalten. Tabletten enthalten ebenfalls Bindemittel, z.B. Magnesiumaluminiumsilikat, Stärke, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Quell- oder Sprengmittel, z.B. Stärke, Agar, Alginsäure oder ein Salz davon, wie Natriumalginat, und/oder Brausemischungen, oder Adsorptionsmittel, Farbstoffe, Geschmackstoffe und Süssstoffe. Suppositorien sind in erster Linie Fettemulsionen oder -suspensionen.

Die vorliegenden pharmazeutischen Präparate, die, wenn erwünscht, weitere pharmakologisch wertvolle Zusatzstoffe enthalten können, werden in an sich bekannter Weise, z.B. mittels konventioneller Mischungs-, Lösungs- oder Lyophilisierverfahren, hergestellt und enthalten etwa 0,1% bis 100%, insbesondere von etwa 1% bis etwa 50%, Lyophilisate bis zu 100%, des Wirkstoffs. Je nach Art der Infektion, Zustand des infizierten Organismus, verwendet man tägliche Dosen von etwa 0,25 bis etwa 2,0 g p.o. zur Behandlung von Warmblütern (Menschen oder Tiere) von etwa 70 kg Gewicht.

Ausgangsmaterialien

Verbindungen der Formel II sind wertvolle Zwischenprodukte und sind ebenfalls Gegenstand der vorliegenden Erfindung. Diese werden beispielsweise hergestellt, indem man eine Verbindung der Formel:

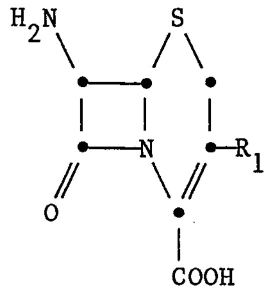

(XIV),

worin die 7ß-Aminogruppe gegebenenfalls geschützt ist und $R_1$ die unter Formel I genannte Bedeutung hat, oder ein reaktionsfähiges, funktionelles Derivat dieser Verbindung gegebenenfalls stufenweise mit einem die Gruppe:

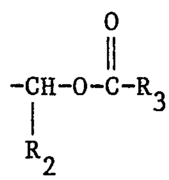

einführenden Veresterungsmittel umsetzt.

Die Herstellung des Ausgangsmaterials der Formel II erfolgt analog den Veresterungsverfahren b), indem eine Verbindung der Formel XIV mit einer Verbindung der Formel IX umgesetzt wird, oder stufenweise, indem eine Verbindung der Formel XIV mit einem Dihalogenid der Formel X oder ein reaktionsfähiges, funktionelles Derivat einer Verbindung der Formel XIV mit einem reaktionsfähigen Derivat des Aldehyds der Formel XI umgesetzt wird. Die Zwischenstufe der Formel:

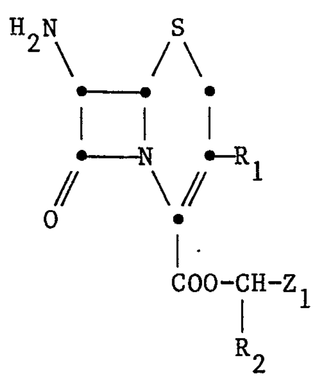

(XV),

welche ebenfalls Gegenstand der vorliegenden Erfindung ist, wird dann anschliessend in situ oder nach Isolierung mit einer Carbonsäure der Formel XIII oder einem Salz dieser Carbonsäure, oder wenn $Z_1$ Hydroxy ist, mit einem reaktionsfähigen, funktionellen Derivat dieser Carbonsäure umgesetzt.

Verbindungen der Formel III sind bekannt oder können, falls sie neu sind, in an sich bekannter Weise hergestellt werden. Wenn in einer Verbindung der Formel III die Gruppe A durch substituiertes Amino substituiertes Methylen darstellt, kann man die Niederalkanoylamino-, Niederalkylsulfonylamino- oder Aminoniederalkylsulfonylaminoderivate durch Acylierung der $\alpha$-Aminoverbindung der Formel III (A = Methylen substituiert durch Amino) mit einer Niederalkancarbonsäure, Niederalkansulfonsäure oder Aminoniederalkansulfonsäure oder einem reaktionsfähigen, funktionellen Derivat davon, z.B. einem Säurechlorid, herstellen. Durch anschliessende Umsetzung mit einem Alkylierungsmittel, z.B. Methyljodid oder Aethyljodid, erhält man Verbindungen der Formel III, worin A durch N-Niederalkyl-N-niederalkanoylamino, N-Niederalkyl-N-niederalkylsulfoynlamino oder N-Niederalkyl-N-aminoniederalkansulfonylamino substituiertes Methylen bedeutet.

Verbindungen der Formel IV sind bekannt oder können, falls sie neu sind, in an sich bekannter Weise, beispielsweise durch Acylierung einer Verbindung der Formel XIV mit einer Verbindung der Formel III analog Verfahren a) hergestellt werden.

Verbindungen der Formel V sind wertvolle Zwischenprodukte und sind ebenfalls Gegenstand der vorliegenden Erfindung. Diese werden beispielsweise hergestellt, indem man in einer Verbindung der Formel:

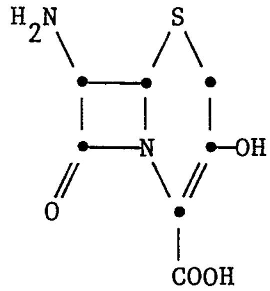

(XVI),

worin die 7ß-Aminogruppe und die 4-Carboxygruppe gegebenenfalls durch eine die Acylierungsreaktion erlaubende Gruppe geschützt sind, die 7ß-Aminogruppe durch Umsetzung mit einem den Acylrest einer Carbonsäure der Formel III einführenden Acylierungsmittel, worin A, X und Y die unter Formel I genannten Bedeutungen haben und die Aminogruppe gegebenenfalls geschützt ist, acyliert und eine erhältliche acylierte Verbindung der Formel:

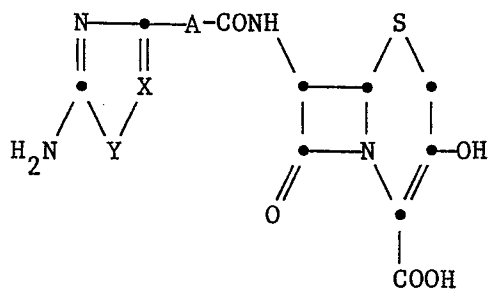

(XVII)

gegebenenfalls in situ mit einem die Gruppe:

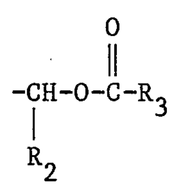

einführenden Veresterungsmittel umsetzt und gegebenenfalls die 3-Hydroxygruppe in eine andere Abgangsgruppe $R_o$ überführt.

Die Acylierung einer Verbindung der Formel XVI mit der Carbonsäure der Formel III oder einem reaktionsfähigen, funktionellen Derivat dieser Carbonsäure, z.B. dem Säurechlorid, erfolgt in der unter Verfahren a) beschriebenen Weise. Die anschliessende Veresterung

erfolgt analog Verfahren b), indem die acylierte Verbindung der Formel XVII mit einer Verbindung der Formel IX umgesetzt wird, oder stufenweise, indem die Verbindung der Formel XVII mit einem Dihalogenid der Formel X oder einem reaktionsfähigen Derivat des Aldehyds der Formel XI umgesetzt wird. Die Ueberführung der 3-Hydroxygruppe in eine andere Abgangsgruppe $R_o$ ist in der Deutschen Offenlegungsschrift 2 332 078 beschrieben und erfolgt z.B. durch 3-Acylierung, z.B. mit dem Acetylrest, z.B. durch Reaktions mit Essigsäureanhydrid.

Die Zwischenstufe der Formel:

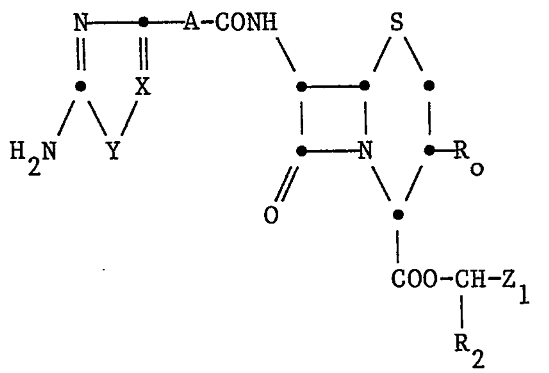

(XVIII),

welche ebenfalls Gegenstand der vorliegenden Erfindung ist, wird dann anschliessend in situ oder nach Isolierung mit einer Carbonsäure der Formel XIII oder einem Salz dieser Carbonsäure, oder wenn $Z_1$ Hydroxy ist, mit einem reaktionsfähigen, funktionellen Derivat dieser Carbonsäure umgesetzt.

Verbindungen der Formel VI sind wertvolle Zwischenprodukte und sind ebenfalls Gegenstand der vorliegenden Erfindung. Diese werden beispielsweise hergestellt, indem man eine Verbindung der Formel:

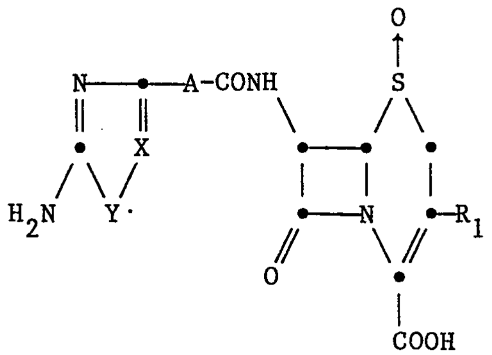

(XIX),

worin X, Y, A und $R_1$ die unter Formel I genannten Bedeutungen haben, oder ein reaktionsfähiges, funktionelles Derivat dieser Verbindung gegebenenfalls stufenweise mit einem die Gruppe:

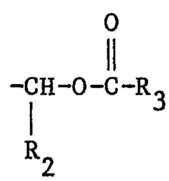

einführenden Veresterungsmittel umsetzt. Die Herstellung des Ausgangsmaterials der Formel VI erfolgt analog dem Veresterungsverfahren b), indem eine Verbindung der Formel XIX mit einer Verbindung der Formel IX umgesetzt wird, oder stufenweise, indem eine Verbindung der Formel XIX mit einem Aldehyd der Formel XI oder eine Verbindung der Formel XIX mit einem reaktionsfähigen Derivat des Aldehyds der Formel XI umgesetzt wird. Die Zwischenstufe der Formel:

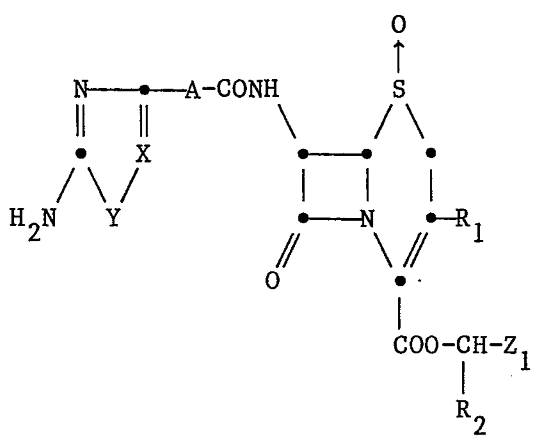

(XX) ,

welche ebenfalls Gegenstand der vorliegenden Erfindung ist, wird dann anschliessend in situ oder nach Isolierung mit einer Carbonsäure der Formel XIII oder einem Salz dieser Carbonsäure oder, wenn $Z_1$ Hydroxy ist, mit einem reaktionsfähigen, funktionellen Derivat dieser Carbonsäure umgesetzt.

Verbindungen der Formel VII sind wertvolle Zwischenprodukte und ebenfalls Gegenstand der vorliegenden Erfindung. Diese werden beispielsweise in an sich bekannter Weise hergestellt, indem man in einer Verbindung der Formel II, worin die 7ß-Aminogruppe und die 4-Carboxygruppe gegebenenfalls durch eine die Acylierungsreaktion erlaubende Gruppe geschützt sind, die 7ß-Aminogruppe durch Umsetzung mit einem den Acylrest einer Carbonsäure der Formel:

$$Hal-CH_2-CO-A-COOH \qquad (XXI),$$

worin Hal Halogen bedeutet und A die unter Formel I genannten Bedeutungen hat und eine in A vorhandene funktionelle Gruppe geschützt ist, einführenden Acylierungsmittel acyliert.

Die Acylierung einer Verbindung der Formel II mit der Carbonsäure der Formel XXI oder einem reaktionsfähigen, funktionellen Derivat dieser Carbonsäure, z.B. dem Säurechlorid, erfolgt analog Verfahren a).

Verbindungen der Formel VIII sind wertvolle Zwischenprodukte und sind ebenfalls Gegenstand der vorliegenden Erfindung. Diese werden beispielsweise in an sich bekannter Weise hergestellt, indem man in einer Verbindung der Formel II, worin die 7ß-Aminogruppe und die 4-Carboxygruppe gegebenenfalls durch eine die Acylierungsreaktion erlaubende Gruppe geschützt ist, die 7ß-Aminogruppe durch Umsetzung mit einem den Acylrest einer Carbonsäure der Formel:

$$X_1-N=C(NH_2)-A-COOH \qquad (XXII)$$

worin $X_1$ Wasserstoff, Halogen oder Hydroxy bedeutet und A die unter Formel I genannte Bedeutung hat und die Aminogruppe und eine in A vorhandene funktionelle Gruppe geschützt ist, einführenden Acylierungsmittel acyliert.

Die Acylierung einer Verbindung der Formel II mit der Carbonsäure der Formel XXII oder einem reaktionsfähigen, funktionellen Derivat dieser Carbonsäure, z.B. dem Säurechlorid, erfolgt analog Verfahren a).

Verbindungen der Formel IX sind bekannt oder lassen sich, falls sie neu sind, durch Umsetzung eines Dihalogenids der Formel X mit einer Carbonsäure der Formel XIII oder einem Salz dieser Carbonsäure, durch Umsetzung eines Aldehyds der Formel XI mit einem reaktionsfähigen, funktionellen Derivat einer Carbonsäure der Formel XIII, z.B. dem Säurechlorid, oder durch Umsetzung eines reaktionsfähigen Derivats des Aldehyds der Formel XI, z.B. eines Acetals, mit einer Carbonsäure der Formel XIII analog Verfahren b) herstellen.

Verbindungen der Formeln XIV und XVI sind bekannt und zum Teil kommerziell erhältlich.

Verbindungen der Formel XV sind bekannt.

Verbindungen der Formel XVII sind bekannt oder können, falls sie neu sind, in an sich bekannter Weise hergestellt werden. Wenn in einer Verbindung der Formel XVI die Gruppe A durch substituiertes Amino substituiertes Methylen darstellt, kann man die Niederalkanoylamino-, Niederalkylsulfonylamino- oder Aminoniederalkylsulfonylaminoderivate durch Acylierung der α-Aminoverbindung der Formel XVII (A = Methylen substituiert durch Amino) mit einer Niederalkancarbonsäure, Niederalkansulfonsäure oder Aminoniederalkansulfonsäure oder einem reaktionsfähigen, funktionellen Derivat davon, z.B. einem Säurechlorid, herstellen. Durch anschliessende Umsetzung mit einem Alkylierungsmittel, z.B. Methyljodid oder Aethyljodid, erhält man Verbindungen der Formel XVII, worin A durch N-Niederalkyl-N-niederalkanoylamino, N-Niederalkyl-N-niederalkylsulfonylamino oder N-Niederalkyl-N-aminoniederalkansulfonylamino substituiertes Methylen bedeutet.

Verbindungen der Formel XIX sind bekannt oder können, falls sie neu sind auf an sich bekannte Weise hergestellt werden, z.B. durch Acylierung einer 1-Oxid-Verbindung der Formel:

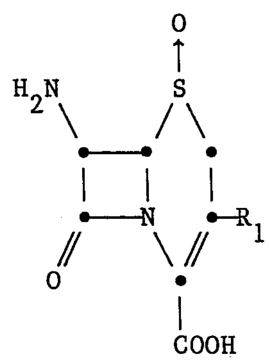

(XXIII)

mit einer Carbonsäure der Formel III analog Verfahren a).

Verbindungen der Formeln XXI, XXII und XXIII sind bekannt.

Die folgenden Beispiele dienen zur Illustration der Erfindung. Temperaturen werden in Grad Celsius angegeben. DC = Dünnschichtchromatogramm.

Beispiel 1: 7ß-[2-(2-Aminothiazol-4-yl)-2-Z-methoxyiminoacetamido]-3-cephem-4-carbonsäure-2-acetoxybenzoyloxymethylester

Eine Suspension von 3,83 g (10 mMol) 7ß-[2-(2-Aminothiazol-4-yl)-2-Z-methoxyiminoacetamido]-3-cephem-4-carbonsäure in 25 ml trockenem N,N-Dimethylformamid wird durch Versetzen mit 1,34 ml (0,9 mMol) 1,5-Diazabicyclo[5.4.0]undec-5-en bei 10° in Lösung gebracht. Man gibt eine Lösung von 4,57 g (20 mMol) 2-Acetoxybenzoyloxymethylchlorid in 5 ml N,N-Dimethylformamid hinzu und rührt 18 Stunden bei 22°. Die erhaltene, dunkelrote, klare Lösung wird bei 0° unter Rühren zu 130 ml 0,3-molarer, wässriger Dikaliumhydrogenphosphat-Lösung gegeben, das Gemisch mit Natriumchlorid gesättigt und dreimal mit je

250 ml kaltem Essigsäureäthylester extrahiert. Die über Natriumsulfat getrockneten und mit Aktivkohle gereinigten, organischen Auszüge werden eingedampft, bis man einen öligen Rückstand erhält. Nach Digerieren des Rückstands in Diäthyläther wird der nicht gelöste Anteil in Essigsäureäthylester gelöst und mit 0,8 ml 12 N wässriger Chlorwasserstoff-Lösung versetzt. Man erhält die Titelverbindung als Hydrochlorid. DC: Rf ca. 0,36 (Aceton-Toluol 6:4, Merck DC-Fertigplatten Kieselgel 60 $F_{254}$); NMR-Spektrum siehe Tabelle.

Beispiel 2: 7ß-[2-(2-Aminothiazol-4-yl)-2-Z-methoxyiminoacetamido]-3-cephem-4-carbonsäure-2-acetoxybenzoyloxymethylester

Eine Suspension von 7,7 g (20 mMol) 7ß-[2-(2-Aminothiazol-4-yl)-2-Z-methoxyiminoacetamido]-3-cephem-4-carbonsäure in 30 ml absolutem Dimethylformamid wird bei 0° bis 5° mit 3,0 ml (20 mMol) 1,5-Diazabicyclo[5.4.0]undec-5-en versetzt und bei dieser Temperatur so lange gerührt (ca. 50 Min.), bis man eine klare Lösung erhält. Nach Zugabe von 1,8 ml (24 mMol) Chlorjodmethan rührt man 17 Stunden bei 22°. Die Lösung, welche den 7ß-[2-(2-Aminothiazol-4-yl)-2-Z-methoxyiminoacetamido]-3-cephem-4-carbonsäurechlormethylester enthält, wird zur Entfernung von überschüssigem Chlorjodmethan im Vakuum etwas eingeengt und anschliessend mit einer Lösung von 5,4 g (30 mMol) 2-Acetoxybenzoesäure und 4,5 ml 1,5-Diazabicyclo[5.4.0]undec-5-en in 25 ml dimethylformamid versetzt. Man lässt 18 Stunden bei 22° stehen und arbeitet analog Beispiel 1 auf. Man erhält ein Rohprodukt, das säulenchromatographisch an Kieselgel gereinigt wird. Aus den mit einer Methylenchlorid-Methanol 9:1-Mischung eluierten Fraktionen erhält man die reine Titelverbindung, die mit dem gemäss Beispiel 1 erhältlichen Produkt identisch ist.

Beispiel 3: In analoger Weise zu Beispiel 1 oder 2 kann man folgende Ester bzw. deren Hydrochloride herstellen (Rf-Werte gelten für das System Aceton-Toluol 6:4, Merck DC-Fertigplatten Kieselgel 60 $F_{254}$; NMR-Werte siehe Tabelle):

a) 7ß-[2-(2-Aminothiazol-4-yl)-2-Z-methoxyiminoacetamido]-3-cephem-4-carbonsäure-4-methoxybenzoyloxymethylester, Rf ca. 0,31;

b) 7ß-[2-(2-Aminothiazol-4-yl)-2-Z-methoxyiminoacetamido]-3-cephem-4-carbonsäure-4-methylbenzoyloxymethylester, Rf ca. 0,40;

c) 7ß-[2-(2-Aminothiazol-4-yl)-2-Z-methoxyiminoacetamido]-3-cephem-4-carbonsäure-4-tert-butylbenzoyloxymethylester, Rf ca. 0,39;

d) 7ß-[2-(2-Aminothiazol-4-yl)-2-Z-methoxyiminoacetamido]-3-cephem-4-carbonsäure-4-benzoyloxymethylester, Rf ca. 0,41;

e) 7ß-[2-(2-Aminothiazol-4-yl)-2-Z-methoxyiminoacetamido]-3-cephem-4-carbonsäure-4-cyanbenzoyloxymethylester, Rf ca. 0,36;

f) 7ß-[2-(2-Aminothiazol-4-yl)-2-Z-methoxyiminoacetamido]-3-cephem-4-carbonsäure-3-chlorbenzoyloxymethylester, Rf ca. 0,42;

g) 7ß-[2-(2-Aminothiazol-4-yl)-2-Z-methoxyiminoacetamido]-3-cephem-4-carbonsäure-4-nitrobenzoyloxymethylester, Rf ca. 0,34;

h) 7ß-[2-(2-Aminothiazol-4-yl)-2-Z-methoxyiminoacetamido]-3-cephem-4-carbonsäure-2-furoyloxymethylester, Rf ca. 0,34;

i) 7ß-[2-(2-Aminothiazol-4-yl)-2-Z-methoxyiminoacetamido]-3-cephem-4-carbonsäure-3,4,5-trimethoxybenzoyloxymethylester, Rf ca. 0,36;

k) 7ß-[2-(2-Aminothiazol-4-yl)-2-Z-methoxyiminoacetamido]-3-cephem-4-carbonsäure-1-benzoyloxyäthylester, Rf ca. 0,39;

l) 7ß-[2-(2-Aminothiazol-4-yl)-2-Z-methoxyiminoacetamido]-3-cephem-4-carbonsäure-4-aminobenzoyloxymethylester, Rf ca. 0,25;

m) 7ß-[2-(2-Aminothiazol-4-yl)-2-Z-methoxyiminoacetamido]-3-cephem-4-carbonsäure-2-acetylbenzoyloxymethylester; Rf ca. 0,35;

n) 7ß-[2-(2-Aminothiazol-4-yl)-2-Z-methoxyiminoacetamido]-3-cephem-4-carbonsäure-3-trifluormethylbenzoyloxymethylester, Rf. ca. 0,45;

o) 7ß-[2-(2-Aminothiazol-4-yl)-2-Z-methoxyiminoacetamido]-3-cephem-4-carbonsäure-4-sulfamoylbenzoyloxymethylester;

p) 7ß-[2-(2-Aminothiazol-4-yl)-2-Z-methoxyiminoacetamido]-3-cephem-4-carbonsäure-4-hydroxybenzoyloxymethylester;

q) 7ß-[2-(2-Aminothiazol-4-yl)-2-Z-methoxyiminoacetamido]-3-cephem-4-carbonsäure-α-benzoyloxybenzylester; Rf: ca. 0.57;

r) 7ß-[2-(2-Aminothiazol-4-yl)-2-Z-methoxyiminoacetamido]-3-cephem-4-carbonsäure-4-di-n-propylsulfamoylbenzoyloxymethylester;
Rf: ca. 0.50;

s) 7ß-[2-(2-Aminothiazol-4-yl)-2-Z-methoxyiminoacetamido]-3-cephem-4-carbonsäure-2-chlorbenzoyloxymethylester; Rf: ca. 0.43;

t) 7ß-[2-(2-Aminothiazol-4-yl)-2-Z-methoxyiminoacetamido]-3-cephem-4-carbonsäure-4-chlorbenzoyloxymethylester; Rf: ca. 0.40;

u) 7ß-[2-(2-Aminothiazol-4-yl)-2-Z-methoxyiminoacetamido]-3-cephem-4-carbonsäure-3,4-diacetoxybenzoyloxymethylester; Rf: ca. 0.37;

v) 7ß-[2-(2-Aminothiazol-4-yl)-2-Z-methoxyiminoacetamido]-3-cephem-4-carbonsäure-4-acetoxybenzoyloxymethylester; Rf: ca. 0.39;

w) 7ß-[2-(2-Aminothiazol-4-yl)-2-Z-methoxyiminoacetamido]-3-cephem-4-carbonsäure-3,5-diacetoxybenzoyloxymethylester; Rf: ca. 0.41;

x) 7ß-[2-(2-Aminothiazol-4-yl)-2-Z-methoxyiminoacetamido]-3-cephem-4-carbonsäure-3-acetoxybenzoyloxymethylester; Rf: ca. 0.34.

Tabelle

Chemische Verschiebungen (in ppm relativ zu Tetramethylsilan) der Protonen in den NMR-Spektren der gemäss Beispiel 1-3 erhaltenen Verbindungen:

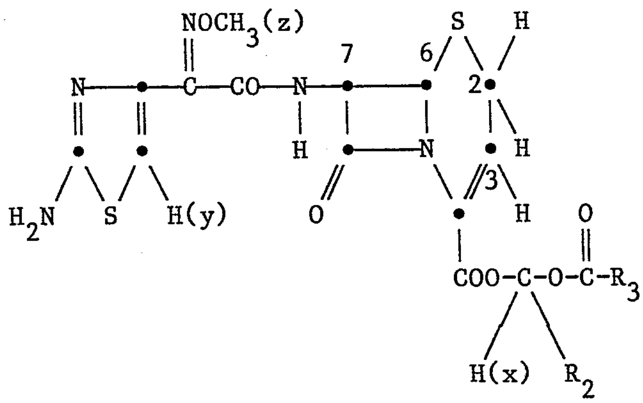

s = Singlett
d = Dublett
dd = Doppeldublett
q = Quadruplett
m = Multiplett
AB = AB-System
J = Kopplungskonstante in Hertz

| Beispiel | $R_2$ | $R_3$ | $H(x)/R_2$ | arom. H | $R_3$ Substituent |
|---|---|---|---|---|---|
| 1 | H | 2-Acetoxyphenyl | 6,08 AB | 7,23-8,05 m | 2,31 s |
| 3a | H | 4-Methoxyphenyl | 6,11 AB | 7,02 d/7,96 d | 3,89 s |
| 3b | H | 4-Methylphenyl | 6,11 AB | 7,37 d/7,89 d | 2,39 s |
| 3c | H | 4-tert-Butylphenyl | 6,12 AB | 7,57 d/7,94 d | 1,31 s |
| 3d | H | Phenyl | 6,14 AB | 7,56-8,08 m | - |
| 3e | H | 4-Cyanphenyl | 6,15 AB | 8,03 d/8,16 d | - |
| 3f | H | 3-Chlorphenyl | 6,12 AB | 7,79 m | - |
| 3g | H | 4-Nitrophenyl | 6,18 AB | 8,23 d/8,40 d | - |
| 3h | H | 2-Furyl | 6,09 AB | 6,74 m/7,44 d/ 8,06 s | - |
| 3i | H | 3,4,5-Trimethoxyphenyl | 6,12 AB | 7,29 s | 3,89 s/ 3,79 s |
| 3k | $CH_3$ | Phenyl | 7,15 q/ 1,66 d | 7,52-8,05 m | - |
| 3l | H | 4-Aminophenyl | 6,03 AB | 6,58 d/7,67 d | - |
| 3m | H | 2-Acetylphenyl | 6,04 AB | 7,71 m | 2,52 s |
| 3n | H | 3-Trifluormethylphenyl | 6,18 AB | 7,76-8,36 m | - |

| Beispiel | $R_2$ | $R_3$ | $H(x)/R_2$ | arom. H | $R_3$ Substituent |
|---|---|---|---|---|---|
| 3o | H | 4-Sulfamoylphenyl | 6,15 AB | 7,97 d/8,15 d | 7,59 s |
| 3p | H | 4-Hydroxyphenyl | 6,06 AB | 6,87 d/7,83 d | 10,45 s |
| 3q | Phenyl | Phenyl | - | 7,48-8,03 m | - |
| 3r | H | 4-N,N-Dipropylsulfamoylphenyl | 6,14 AB | 7,96 d/8,15 d | 0,80t/ 1,47m/ 3,06 t |
| 3s | H | 2-Chlorphenyl | 6,13 AB | 7,43-7,94 m | - |
| 3t | H | 4-Chlorphenyl | 6,13 AB | 7,64 d/8,00 d | - |
| 3u | H | 3,4-Diacetoxyphenyl | 6,15 AB | 7,47-8,02 m | 2,33 s |
| 3v | H | 4-Acetoxyphenyl | 6,14 AB | 7,34 d/8,05 d | 2,32 s |
| 3w | H | 3,5-Diacetoxyphenyl | 6,14 AB | 7,38-7,72 m | 2,31 s |
| 3x | H | 3-Acetoxyphenyl | 6,15 AB | 7,44-7,98 m | 2,31 s |

Banden der übrigen H in folgenden Bereichen:

H-2: 3,65-3,71 m, H-3: 6,62-6,72 m, H-6: 5,15-5,20 d (J = 5)
H-7: 5,87-5,90 dd (J = 5 und 8), NH-7: 9,59-9,83 d (J =8),
H(y): 6,76-6,95 s, H(z): 3,85-3,99 s.

Beispiel 4: Tabletten, enthaltend 250 mg 7ß-[2-(2-Aminothiazol-4-yl)-2-Z-methoxyiminoacetamido]-3-cephem-4-carbonsäure-2-acetoxybenzoyloxymethylesterhydrochlorid als Wirkstoff werden wie folgt hergestellt:

Zusammensetzung (für 1 Tablette)

| | |
|---|---|
| Wirkstoff | 250 mg |
| Mikrokristalline Cellulose | 80 mg |
| Natriumcarboxymethylstärke | 10 mg |
| Magnesiumstearat | 3 mg |
| Talk | 7 mg |
| | 350 mg |

Der Wirkstoff wird mit den Zusatzstoffen homogen vermischt und zu Tabletten gepresst. Zur Herstellung von Filmdragées werden die Tabletten je mit 1 mg wässrigem Lack überzogen. Anstelle von Natriumcarboxymethylstärke kann auch Natriumcarboxymethylcellulose eingesetzt werden.

Beispiel 5: Kapseln, enthaltend 0,250 g 7ß-[2-(2-Aminothiazol-4-yl)-2-Z-methoxyiminoacetamido]-3-cephem-4-carbonsäure-2-acetoxybenzoyloxymethylesterhydrochlorid als Wirkstoff, werden wie folgt hergestellt:

Zusammensetzung (für 100 000 Kapseln)

| | |
|---|---|
| Wirkstoff | 25 000 g |
| Weizenstärke | 2 500 g |
| Magnesiumstearat | 1 000 g |

Der Wirkstoff, die Weizenstärke und das Magnesiumstearat werden gut miteinander vermischt und in Kapseln Nr. 1 abgefüllt.

Beispiel 6: Kapseln, enthaltend 0,5 g 7ß-[2-(2-Aminothiazol-4-yl)-2-Z-methoxyiminoacetamido]-3-cephem-4-carbonsäure-2-acetoxybenzoyloxymethylesterhydrochlorid als Wirkstoff, werden wie folgt hergestellt:

<u>Zusammensetzung</u> (für 2000 Kapseln)

| | |
|---|---|
| Wirkstoff | 1000,00 g |
| Polyvinylpyrrolidon | 15,00 g |
| Maisstärke | 115,00 g |
| Magnesiumstearat | 20,00 g |

Man versetzt den Wirkstoff mit 300 ml einer Lösung des Polyvinylpyrrolidons in 95%igem Aethanol, treibt das Gemisch durch ein Sieb mit 3 mm Maschenweite und trocknet das Granulat unter vermindertem Druck bei 40-50°. Man siebt das Granulat durch ein Sieb mit 0,8 mm Maschenweite, gibt die Maisstärke und das Magnesiumstearat zu, vermischt und füllt das Gemisch in Steckkapseln (Grösse 0) ab.

Patentansprüche für die Vertragsstaaten: GB, BE, DE, FR, IT, SE, LU, NL, CH, LI

1. Verbindungen der Formel:

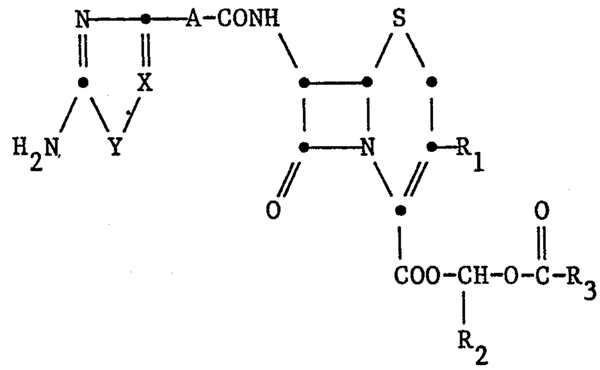

(I) ,

worin X Stickstoff oder die Gruppe $-\overset{\|}{C}-H$, Y Sauerstoff, Schwefel oder die Gruppen >N-H oder >N-Niederalkyl, A Methylen oder durch Amino, substituiertes Amino, Hydroxy, Carboxy, Hydroxyimino oder veräthertes oder verestertes Hydroxyimino substituiertes Methylen, $R_1$ Wasserstoff, Halogen, Niederalkyl oder Niederalkoxy, $R_2$ Wasserstoff, Niederalkyl, Phenyl oder substituiertes Phenyl und $R_3$ unsubstituiertes oder substituiertes, aromatisches, fünf- oder sechsgliedriges Heterocyclyl, Phenyl oder substituiertes Phenyl bedeuten, Stereoisomere, Mischungen von diesen Stereoisomeren und Salze von diesen Verbindungen.

2. Verbindungen gemäss Anspruch 1 der Formel I, worin X Stickstoff oder die Gruppe $-\overset{\|}{C}-H$, Y Sauerstoff oder Schwefel, A durch Amino, Niederalkylsulfonylamino, N,N-Diniederalkylsulfonylamino, N-Aminoniederalkylsulfonyl-N-niederalkylamino, Hydroxyimino, Niederalkoxyimino oder Carboxyniederalkoxyimino substituiertes Methylen, $R_1$ Wasserstoff, Halogen, Niederalkyl oder Niederalkoxy, $R_2$ Wasserstoff, Niederalkyl, Phenyl, Niederalkyl-, Diniederalkyl- oder Triniederalkylphenyl, Hydroxy-, Dihydroxy- oder Trihydroxyphenyl, Hydroxyniederalkylphenyl, Niederalkoxy-, Diniederalkoxy- oder Triniederalkoxyphenyl, Hydroxy-niederalkoxyphenyl, Hydroxy-diniederalkoxyphenyl, Niederalkanoylphenyl, Niederalkanoyloxyphenyl, Halogenphenyl, Nitrophenyl, Cyanphenyl, Amino- oder Diaminophenyl, Aminohydroxyphenyl, Niederalkylaminophenyl, Diniederalkylaminophenyl, Niederalkanoylaminophenyl, Sulfamoylphenyl, Halogenniederalkylphenyl oder Aminoniederalkylphenyl und $R_3$ Pyrrolyl, Niederalkylpyrrolyl, Imidazolyl, Niederalkylimidazolyl, Triazolyl, Niederalkyltriazolyl, Tetrazolyl, Niederalkyltetrazolyl, Thienyl, Thiazolyl, Thiadiazolyl, Furyl, Pyridyl, Hydroxypyridyl, Dihydroxypyridyl, Aminopyridyl, Dihydroxypyrimidinyl, Pyrazinyl, Aminopyrazinyl, oder Pyronyl, Phenyl oder substituiertes Phenyl mit den unter $R_2$ genannten Substituenten bedeuten, und ihre pharmazeutisch annehmbaren Salze.

3. Verbindungen gemäss Anspruch 2 der Formel I, worin X Stickstoff oder die Gruppe $-\overset{\|}{C}-H$, Y Sauerstoff oder Schwefel, A durch Hydroxyimino, Niederalkoxyimino oder Carboxyniederalkoxyimino, substituiertes Methylen, $R_1$ Wasserstoff, Halogen oder Niederalkoxy, $R_2$ Wasserstoff, Niederalkyl, Phenyl, Niederalkylphenyl, Hydroxy-, Dihydroxy- oder Trihydroxyphenyl, Niederalkoxy-, Diniederalkoxy- oder Triniederalkoxyphenyl, Hydroxy-niederalkoxyphenyl, Niederalkanoylphenyl, Niederalkanoyloxyphenyl, Halogenphenyl, Nitrophenyl, Niederalkanoylaminophenyl, Halogenniederalkylphenyl, oder Aminoniederalkylphenyl, und $R_3$ Pyrrolyl, Niederalkylpyrrolyl, Imidazolyl, Niederalkylimidazolyl, Triazolyl, Niederalkyltriazolyl, Tetrazolyl, Niederalkyltetrazolyl, Thienyl, Thiazolyl, Thiadiazolyl, Furyl, Pyridyl, Hydroxypyridyl, Dihydroxypyridyl, Aminopyridyl, Dihydroxypyrimidinyl, Pyrazinyl, Aminopyrazin-2-yl, Pyronyl, Phenyl, Niederalkyl-, Diniederalkyl- oder Triniederalkylphenyl, Hydroxy-, Dihydroxy- oder Trihydroxyphenyl, Hydroxy-niederalkylphenyl, Niederalkoxy-, Diniederalkoxy- oder Triniederalkoxyphenyl, 4-Methoxy-, 3,4-Dimethoxy-, 2,3-Dimethoxy-, 2,4-Dimethoxy- oder 3,4,5-Trimethoxyphenyl, Hydroxy-niederalkoxyphenyl, Hydroxy-diniederalkoxyphenyl, Niederalkanoylphenyl, Niederalkanoyloxyphenyl, Halogenphenyl, Nitrophenyl, Cyanphenyl, Amino- oder Diaminophenyl, Aminohydroxyphenyl, Niederalkylaminophenyl, Diniederalkylaminophenyl, Niederalkanoylaminophenyl, Sulfamoylphenyl, Halogenniederalkylphenyl oder Aminoniederalkylphenyl, bedeuten und ihre pharmazeutisch annehmbaren Salze.

4. Verbindungen gemäss Anspruch 3 der Formel I, worin X die Gruppe
||
-C-H, Y Schwefel, A durch Niederalkoxyimino substituiertes Methylen, $R_1$ Wasserstoff, $R_2$ Wasserstoff oder Niederalkyl, und $R_3$ Furyl, Phenyl, Niederalkyl-, Diniederalkyl- oder Triniederalkylphenyl, Hydroxy-, Dihydroxy- oder Trihydroxyphenyl, Hydroxy-niederalkylphenyl, Niederalkoxy-, Diniederalkoxy- oder Triniederalkoxyphenyl, Hydroxy-niederalkoxyphenyl, Niederalkanoylphenyl, Niederalkanoyloxyphenyl, Halogenphenyl, Nitrophenyl, Cyanphenyl, Amino- oder Diaminophenyl, Amino-hydroxyphenyl, Niederalkylaminophenyl, Diniederalkylaminophenyl, Niederalkanoylaminophenyl, Halogenniederalkylphenyl, oder Aminoniederalkylphenyl bedeuten, und ihre pharmazeutisch annehmbaren Salze.

5. Verbindungen gemäss Anspruch 4 der Formel I, worin X die Gruppe
||
-C-H, Y Schwefel, A durch Methoxyimino substituiertes Methylen, $R_1$ Wasserstoff, $R_2$ Wasserstoff oder Methyl und $R_3$ Fur-2- oder -3-yl, Phenyl, 2-, 3- oder 4-Methyl-, 2-, 3- oder 4-tert-Butyl-, 3,4-Dimethyl-, 2,3-Dimethyl-, 2,4-Dimethyl- oder 2,4,6-Trimethylphenyl, 4-Hydroxy-, 2,4-Dihydroxy-, 2,3,4-Trihydroxy-, 2,4,6-Trihydroxy-oder 3,4,5-Trihydroxyphenyl, 2-Hydroxy-3-methylphenyl, 4-Methoxy-, 3,4-Dimethoxy-, 2,3-Dimethoxy-, 2,4-Dimethoxy- oder 3,4,5-Trimethoxyphenyl, 4-Hydroxy-3-methoxyphenyl, 3-Hydroxy-2,4-dimethoxyphenyl, 2-, 3- oder 4-Acetylphenyl, 2-, 3-oder 4-Acetoxyphenyl, 2-, 3- oder 4-Fluor- oder 2-, 3- oder 4-Chlorphenyl, 2-, 3- oder 4-Nitrophenyl, 2-, 3- oder 4-Cyanphenyl, 2-, 3- oder 4-Aminophenyl oder 3,5-Diaminophenyl, 3-Amino-4-hydroxy-oder 4-Amino-2-hydroxyphenyl, 2- oder 4-Methylaminophenyl, 2-, 3-oder 4-Dimethylaminophenyl, 2-, 3- oder 4-Sulfamoylphenyl, 2-, 3-oder 4-Trifluormethylphenyl oder 2-, 3- oder 4-Aminomethylphenyl bedeuten, und ihre pharmazeutisch annehmbaren Salze.

6. 7ß-[2-(2-Aminothiazol-4-yl)-2-Z-methoxyiminoacetamido]-3-cephem-4-carbonsäure-2-acetoxybenzoyloxymethylester gemäss Anspruch 1.

7. 7ß-[2-(2-Aminothiazol-4-yl)-2-Z-methoxyiminoacetamido]-3-cephem-4-carbonsäure-4-methylbenzoyloxymethylester gemäss Anspruch 1.

8. 7ß-[2-(2-Aminothiazol-4-yl)-2-Z-methoxyiminoacetamido]-3-cephem-4-carbonsäure-4-tert-butylbenzoyloxymethylester gemäss Anspruch 1.

9. Pharmazeutisch annehmbare Salze von Verbindungen gemäss einem der Ansprüche 6 bis 8.

10. Verfahren zur Herstellung von Verbindungen der Formel I und Salzen dieser Verbindungen, dadurch gekennzeichnet, dass man
a) in einer Verbindung der Formel:

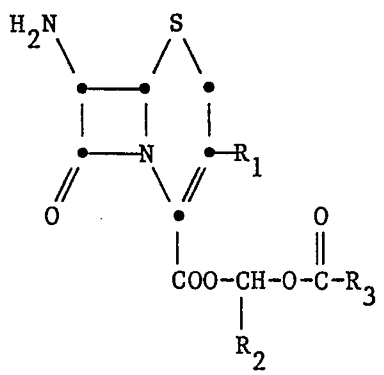

(II),

worin $R_1$, $R_2$ und $R_3$ die unter Formel I genannten Bedeutungen haben, die 7ß-Aminogruppe gegebenenfalls durch eine die Acylierungsreaktion erlaubende Gruppe geschützt ist und eine in $R_2$ oder $R_3$ vorhandene funktionelle Gruppe geschützt ist, die 7ß-Aminogruppe durch Umsetzung mit einem den Acylrest einer Carbonsäure der Formel:

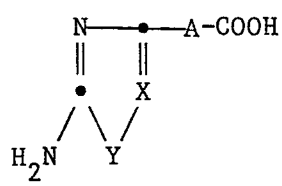

(III)

einführenden Acylierungsmittel, worin A, X und Y und die unter Formel I genannten Bedeutungen haben und die Aminogruppe gegebenenfalls geschützt ist, acyliert, oder

b) eine Verbindung der Formel:

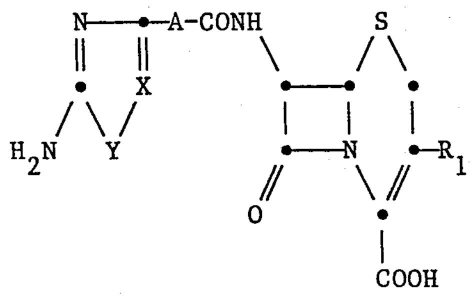

(IV),

worin A, X, Y und $R_1$ die unter Formel I genannten Bedeutungen haben, die Aminogruppe gegebenenfalls und eine in A vorhandene funktionelle Gruppe geschützt ist, oder ein Salz davon oder ein reaktionsfähiges, funktionelles Derivat dieser Verbindung, gegebenenfalls stufenweise mit einem die Gruppe:

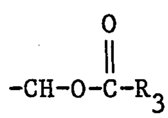

einführenden Veresterungsmittel umsetzt, oder

c) zur Herstellung einer Verbindung der Formel I, worin X, Y, A, $R_2$ und $R_3$ die unter Formel I genannten Bedeutungen haben und $R_1$ Wasserstoff bedeutet, in einer Verbindung der Formel:

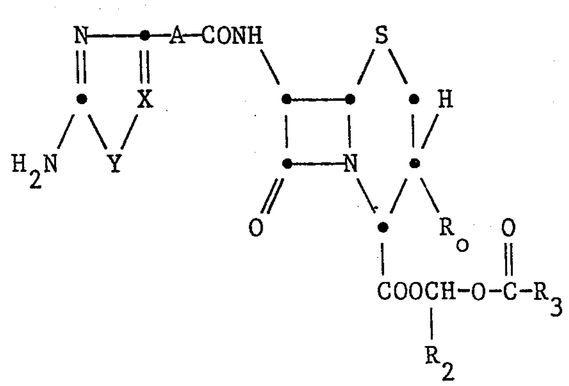

(V),

worin X, Y, A, $R_2$ und $R_3$ die unter Formel I genannten Bedeutungen haben, die Aminogruppe gegebenenfalls und eine in A, $R_2$ oder $R_3$ vorhandene funktionelle Gruppe geschützt ist, $R_o$ eine Abgangsgruppe ist, $R_o$ abspaltet oder

d) ein 1-Oxid der Formel:

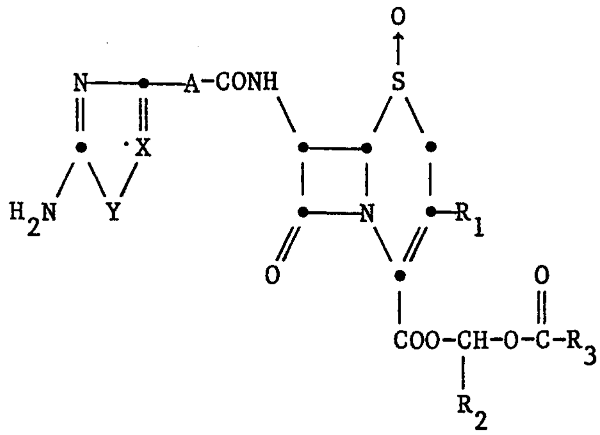

(VI),

worin X, Y, A, $R_1$, $R_2$ und $R_3$ die unter Formel I genannten Bedeutungen haben, die Aminogruppe und eine in A, $R_2$ oder $R_3$ vorhandene funktionelle Gruppe gegebenenfalls geschützt ist, mit einem geeigneten Reduktionsmittel in das 1-Sulfid überführt, oder

e) zur Herstellung von Verbindungen der Formel I, worin X die Gruppe =C-H und Y Sauerstoff oder Schwefel bedeuten, A, $R_1$, $R_2$ und $R_3$ die unter Formel I genannten Bedeutungen haben, eine Verbindung der Formel:

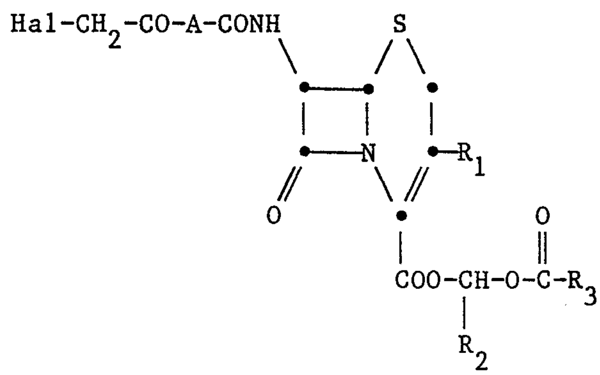

(VII),

worin Hal Halogen bedeutet, A, $R_1$, $R_2$ und $R_3$ die unter Formel I genannten Bedeutungen haben, die Aminogruppe und eine in A, $R_2$ oder $R_3$ vorhandene funktionelle Gruppen gegebenenfalls geschützt ist, mit Harnstoff bzw. Thioharnstoff kondensiert, oder

f) zur Herstellung einer Verbindung der Formel I, worin X Stickstoff und Y Schwefel bedeuten, A, $R_1$, $R_2$ und $R_3$ die unter Formel I genannten Bedeutungen haben, eine Verbindung der Formel:

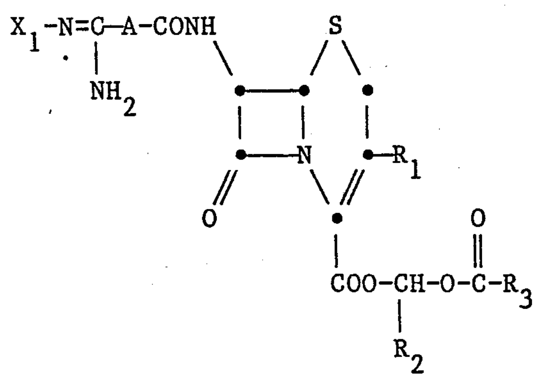

(VIII),

worin $X_1$ Wasserstoff, Halogen oder Hydroxy bedeutet, A, $R_1$, $R_2$ und $R_3$ die unter Formel I genannten Bedeutungen haben, die Aminogruppe und eine in A, $R_2$ oder $R_3$ vorhandene funktionelle Gruppe gegebenenfalls geschützt ist, mit einem Salz der Rhodanwasserstoffsäure oder, falls $X_1$ Wasserstoff ist, mit Dirhodan umsetzt und in einer erhältlichen Verbindung die Aminoschutzgruppe(n) und vorhandene andere Schutzgruppen abspaltet und, wenn erwünscht, eine erhältliche Verbindung in ein Salz oder ein erhältliches Salz in die freie Verbindung oder in ein anderes Salz überführt.

11. Die nach dem Verfahren gemäss Anspruch 10 erhältlichen Verbindungen.

12. Pharmazeutische Präparate enthaltend Verbindungen der Formel I, Hydrate oder pharmazeutisch verwendbare Salze von Verbindungen der Formel I mit salzbildenden Gruppen.

13. Verbindungen der Formel I, Hydrate oder pharmazeutisch verwendbare Salze von Verbindungen der Formel I zur Anwendung in einem Verfahren zur therapeutischen Behandlung des menschlischen oder tierischen Körpers.

14. Verbindungen der Formel I, Hydrate oder pharmazeutisch verwendbare Salze von Verbindungen der Formel I als antibiotisch wirksame Mittel.

15. Verwendung von Verbindungen der Formel I, Hydrate oder pharmazeutisch verwendbaren Salze von Verbindungen der Formel I zur Herstellung von pharmazeutischen Präparaten.

16. Verbindungen der Formel

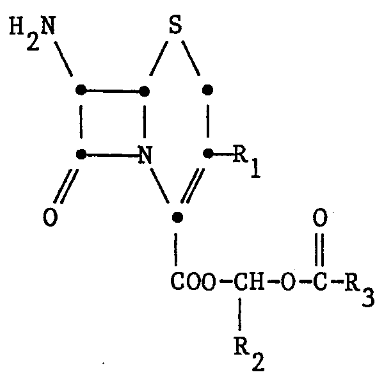

(II),

worin $R_1$, $R_2$ und $R_3$ die unter Formel I genannten Bedeutungen haben.

17. Verfahren zur Herstellung von Verbindungen der Formel II gemäss Anspruch 16, dadurch gekennzeichnet, dass man eine Verbindung der Formel:

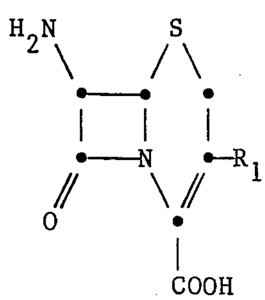

(XIV),

worin die 7ß-Aminogruppe gegebenenfalls geschützt ist und $R_1$ die unter Formel I genannte Bedeutung hat, oder ein reaktionsfähiges, funktionelles Derivat dieser Verbindung gegebenenfalls stufenweise, mit einem die Gruppe:

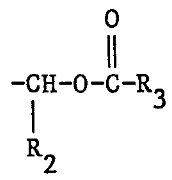

einführenden Veresterungsmittel umsetzt.

18. Verbindungen der Formel:

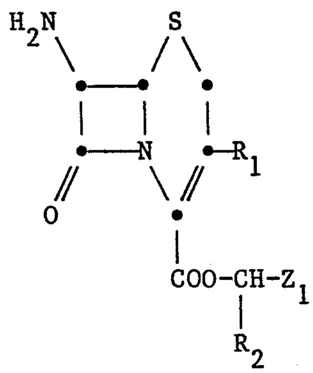

(XV),

worin $R_1$ und $R_2$ die unter Formel I genannten Bedeutungen haben und $Z_1$ Hydroxy oder eine reaktionsfähige, veresterte Hydroxygruppe ist.

19. Verbindungen der Formel:

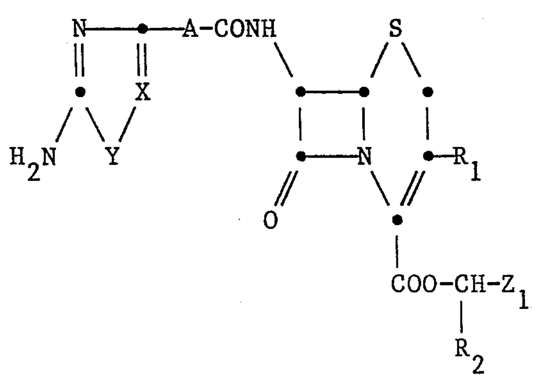

(XII),

worin $Z_1$ Hydroxy oder eine reaktionsfähige, veresterte Hydroxygruppe ist und X, Y, A, $R_1$ und $R_2$ die unter Formel I genannten Bedeutungen haben.

20. Verbindungen der Formel:

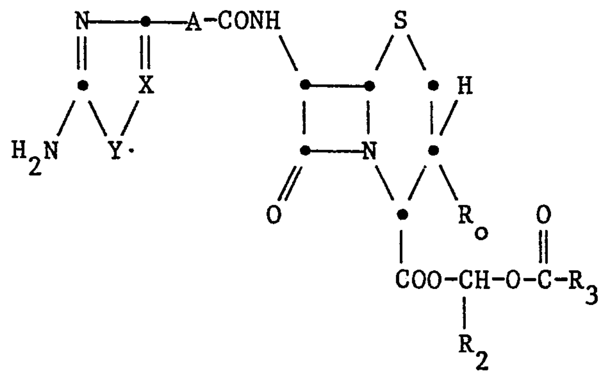

(V),

worin X, Y, A, $R_2$ und $R_3$ die unter Formel I genannten Bedeutungen haben und $R_o$ eine Abgangsgruppe ist.

21. Verbindungen der Formel:

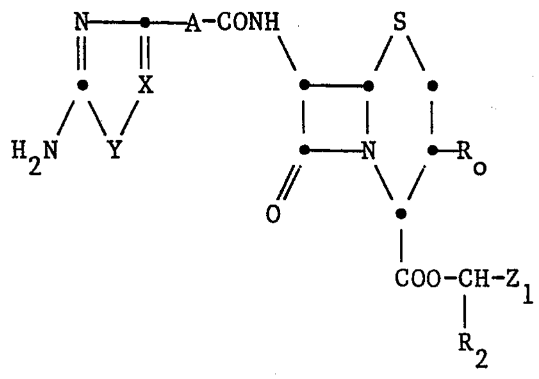

(XVIII),

worin X, Y, A und $R_2$ die unter Formel I genannten Bedeutungen haben, $Z_1$ Hydroxy oder eine reaktionsfähige, veresterte Hydroxygruppe ist und $R_o$ eine Abgangsgruppe darstellt.

22. Verbindungen der Formel:

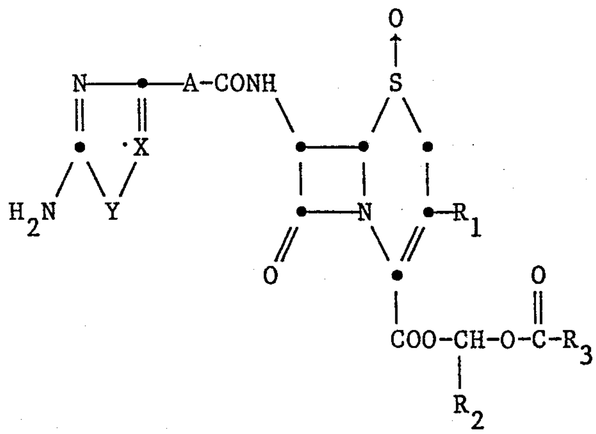

(VI),

worin X, Y, A, $R_1$, $R_2$ und $R_3$ die unter Formel I genannten Bedeutungen haben.

23. Verbindungen der Formel:

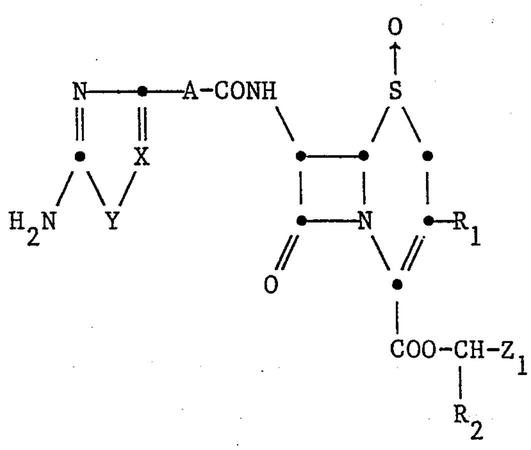

(XX),

worin X, Y, A, $R_1$ und $R_2$ die unter Formel I genannten Bedeutungen haben und $Z_1$ Hydroxy oder eine reaktionsfähige, veresterte Hydroxygruppe ist.

24. Verbindungen der Formel:

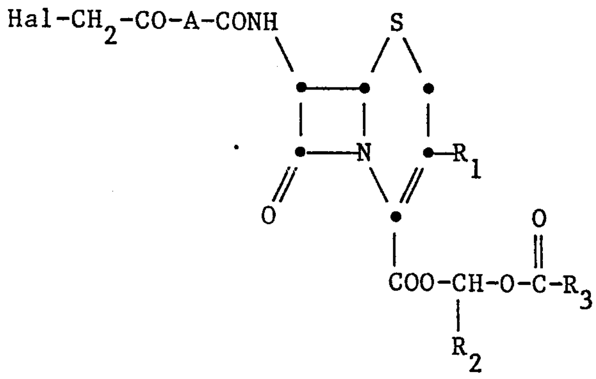

(VII),

worin Hal Halogen bedeutet und A, $R_1$, $R_2$ und $R_3$ die unter Formel I genannten Bedeutungen haben.

25. Verbindungen der Formel:

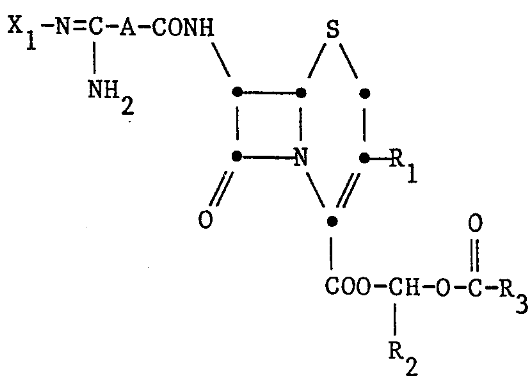

(VIII),

worin $X_1$ Wasserstoff, Halogen oder Hydroxy bedeutet und A, $R_1$, $R_2$ und $R_3$ die unter Formel I genannten Bedeutungen haben.

FO 7.4/RS/gs*

## Patentansprüche für den Vertragsstaat AT

1. Verfahren zur Herstellung von Verbindungen der Formel:

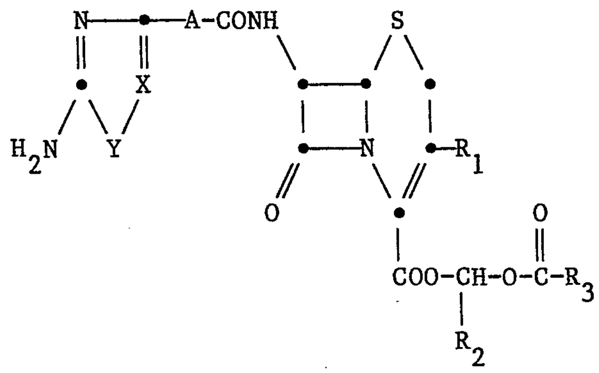

(I) ,

worin X Stickstoff oder die Gruppe $=C-H$, Y Sauerstoff, Schwefel oder die Gruppen $>N-H$ oder $>N$-Niederalkyl, A Methylen oder durch Amino, substituiertes Amino, Hydroxy, Carboxy, Hydroxyimino oder veräthertes oder verestertes Hydroxyimino substituiertes Methylen, $R_1$ Wasserstoff, Halogen, Niederalkyl oder Niederalkoxy, $R_2$ Wasserstoff, Niederalkyl, Phenyl oder substituiertes Phenyl und $R_3$ unsubstituiertes oder substituiertes, aromatisches, fünf- oder sechsgliedriges Heterocyclyl, Phenyl oder substituiertes Phenyl bedeuten, Stereoisomeren, Mischungen von diesen Stereoisomeren und Salzen von diesen Verbindungen, dadurch gekennzeichnet, dass man

a) in einer Verbindung der Formel:

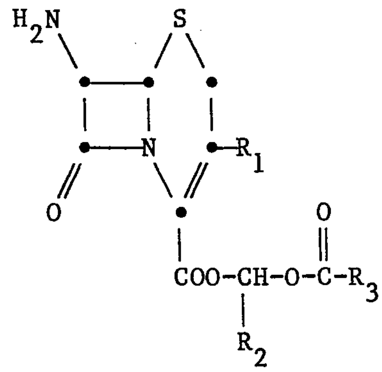

(II),

worin $R_1$, $R_2$ und $R_3$ die unter Formel I genannten Bedeutungen haben, die 7ß-Aminogruppe gegebenenfalls durch eine die Acylierungsreaktion erlaubende Gruppe geschützt ist und eine in $R_2$ oder $R_3$ vorhandene funktionelle Gruppe geschützt ist, die 7ß-Aminogruppe durch Umsetzung mit einem den Acylrest einer Carbonsäure der Formel:

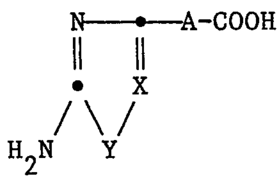

(III)

einführenden Acylierungsmittel, worin A, X und Y die unter Formel I genannten Bedeutungen haben und die Aminogruppe gegebenenfalls geschützt ist, acyliert, oder

b) eine Verbindung der Formel:

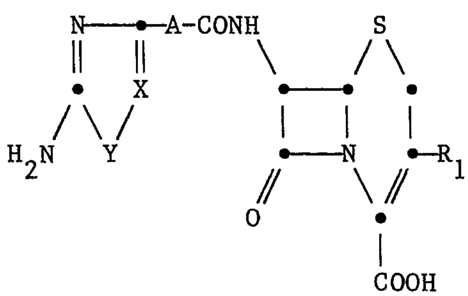

(IV),

worin A, X, Y und $R_1$ die unter Formel I genannten Bedeutungen haben, die Aminogruppe gegebenenfalls und eine in A vorhandene funktionelle Gruppe geschützt ist, oder ein Salz davon oder ein reaktionsfähiges, funktionelles Derivat dieser Verbindung, gegebenenfalls stufenweise mit einem die Gruppe:

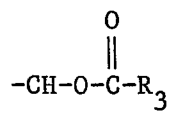

einführenden Veresterungsmittel umsetzt, oder

c) zur Herstellung einer Verbindung der Formel I, worin X, Y, A, $R_2$ und $R_3$ die unter Formel I genannten Bedeutungen haben und $R_1$ Wasserstoff bedeutet, eine Verbindung der Formel:

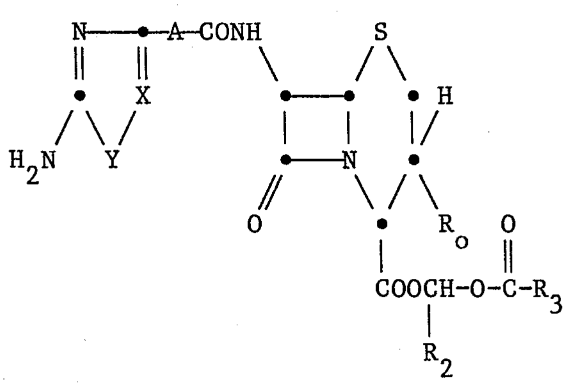

(V),

worin X, Y, A, $R_2$ und $R_3$ die unter Formel I genannten Bedeutungen haben, die Aminogruppe gegebenenfalls und eine in A, $R_2$ oder $R_3$ vorhandene funktionelle Gruppe geschützt ist, $R_o$ eine Abgangsgruppe ist, $R_o$ abspaltet oder

d) ein 1-Oxid der Formel:

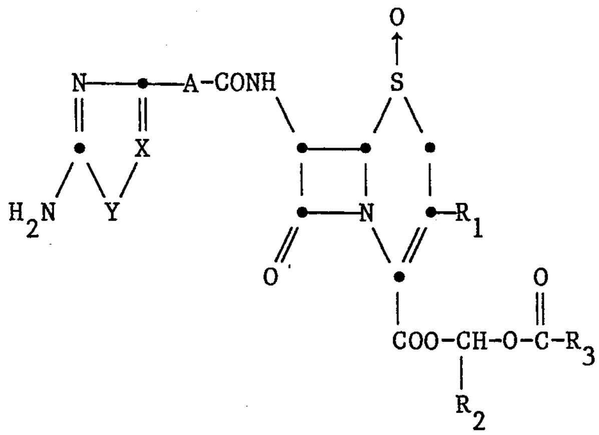

(VI),

worin X, Y, A, $R_1$, $R_2$ und $R_3$ die unter Formel I genannten Bedeutungen haben, die Aminogruppe und eine in A, $R_2$ oder $R_3$ vorhandene funktionelle Gruppe gegebenenfalls geschützt ist, mit einem geeigneten Reduktionsmittel in das 1-Sulfid überführt,

e) zur Herstellung von Verbindungen der Formel I, worin X die Gruppe =C-H und Y Sauerstoff oder Schwefel bedeuten, A, $R_1$, $R_2$ und $R_3$ die unter Formel I genannten Bedeutungen haben, in einer Verbindung der Formel:

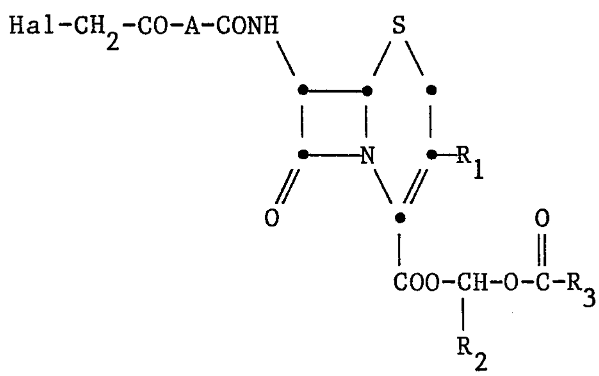

(VII),

worin Hal Halogen bedeutet, A, $R_1$, $R_2$ und $R_3$ die unter Formel I genannten Bedeutungen haben, die Aminogruppe und eine in A, $R_2$ oder $R_3$ vorhandene funktionelle Gruppen gegebenenfalls geschützt ist, mit Harnstoff bzw. Thioharnstoff kondensiert, oder

f) zur Herstellung einer Verbindung der Formel I, worin X Stickstoff und Y Schwefel bedeuten, A, $R_1$, $R_2$ und $R_3$ die unter Formel I genannten Bedeutungen haben, eine Verbindung der Formel:

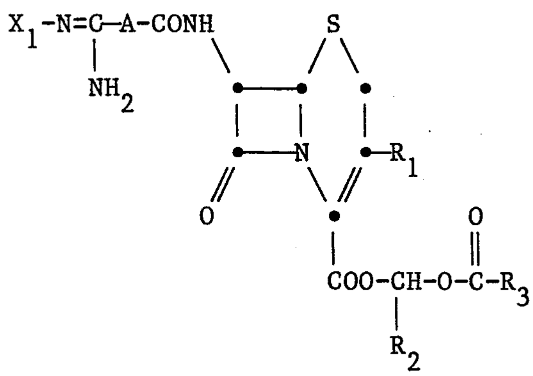

(VIII),

worin $X_1$ Wasserstoff, Halogen oder Hydroxy bedeutet, A, $R_1$, $R_2$ und $R_3$ die unter Formel I genannten Bedeutungen haben, die Aminogruppe und eine in A, $R_2$ oder $R_3$ vorhandene funktionelle Gruppe gegebenenfalls geschützt ist, mit einem Salz der Rhodanwasserstoffsäure oder, falls $X_1$ Wasserstoff ist, mit Dirhodan umsetzt und in einer erhältlichen Verbindung die Aminoschutzgruppe(n) und vorhandene andere Schutzgruppen abspaltet und, wenn erwünscht, eine erhältliche Verbindung in ein Salz oder ein erhältliches Salz in die freie Verbindung oder in ein anderes Salz überführt.

2. Verfahren zur Herstellung von Verbindungen gemäss Anspruch 1 der Formel I, worin X Stickstoff oder die Gruppe $\overset{\|}{-C}-H$, Y Sauerstoff oder Schwefel, A durch Amino, Niederalkylsulfonylamino, N,N-Diniederalkylsulfonylamino, N-Aminoniederalkylsulfonyl-N-niederalkylamino, Hydroxyimino, Niederalkoxyimino oder Carboxyniederalkoxyimino substituiertes Methylen, $R_1$ Wasserstoff, Halogen, Niederalkyl oder Niederalkoxy, $R_2$ Wasserstoff, Niederalkyl, Phenyl, Niederalkyl-, Diniederalkyl- oder Triniederalkylphenyl, Hydroxy-, Dihydroxy- oder Trihydroxyphenyl, Hydroxy-niederalkylphenyl, Niederalkoxy-, Diniederalkoxy- oder Triniederalkoxyphenyl, - Hydroxy-niederalkoxyphenyl, Hydroxy-diniederalkoxyphenyl, Niederalkanoylphenyl, Niederalkanoyloxyphenyl, Halogenphenyl, Nitrophenyl, Cyanphenyl, Amino- oder Diaminophenyl, Amino-hydroxyphenyl, Niederalkylaminophenyl, Diniederalkylaminophenyl, Niederalkanoylaminophenyl, Sulfamoylphenyl, Halogenniederalkylphenyl, oder Aminoniederalkylphenyl und $R_3$ Pyrrolyl, Niederalkylpyrrolyl, Imidazolyl, Niederalkylimidazolyl, Triazolyl, Niederalkyltriazolyl, Tetrazolyl, Niederalkyltetrazolyl, Thienyl, Thiazolyl, Thiadiazolyl, Furyl, Pyridyl, Hydroxypyridyl, Dihydroxypyridyl, Aminopyridyl, Dihydroxypyrimidinyl, Pyrazinyl, Aminopyrazinyl oder Pyronyl, Phenyl oder substituiertes Phenyl mit den unter $R_2$ genannten Substituenten bedeuten, und ihren pharmazeutisch annehmbaren Salzen, dadurch gekennzeichnet, dass man die in Anspruch 1 genannten Massnahmen durchführt.

3. Verfahren zur Herstellung von Verbindungen gemäss Anspruch 2 der Formel I, worin X Stickstoff oder die Gruppe

$$\overset{\|}{-C}-H$$

, Y Sauerstoff oder Schwefel, A durch Hydroxyimino, Niederalkoxyimino oder Carboxyniederalkoxyimino substituiertes Methylen, $R_1$ Wasserstoff, Halogen oder Niederalkoxy, $R_2$ Wasserstoff, Niederalkyl, Phenyl, Niederalkylphenyl, Hydroxy-, Dihydroxy- oder Trihydroxyphenyl, Niederalkoxy-, Diniederalkoxy- oder Triniederalkoxyphenyl, -Hydroxy-niederalkoxyphenyl, Niederalkanoylphenyl, Niederalkanoyloxyphenyl, Halogenphenyl, Nitrophenyl, Niederalkanoylaminophenyl, Halogenniederalkylphenyl, oder Aminoniederalkylphenyl, und $R_3$ Pyrrolyl, Niederalkylpyrrolyl, Imidazolyl, Niederalkylimidazolyl, Triazolyl, Niederalkyltriazolyl, Tetrazolyl, Niederalkyltetrazolyl, Thienyl, Thiazolyl, Thiadiazolyl, Furyl, Pyridyl, Hydroxypyridyl, Dihydroxypyridyl, Aminopyridyl, Dihydroxypyrimidinyl, Pyrazinyl, Aminopyrazin-2-yl, Pyronyl, Phenyl, Niederalkyl-, Diniederalkyl-oder Triniederalkylphenyl, Hydroxy-, Dihydroxy- oder Trihydroxyphenyl, Hydroxy-niederalkylphenyl, Niederalkoxy-, Diniederalkoxy-oder Triniederalkoxyphenyl, 4-Methoxy-, 3,4-Dimethoxy-, 2,3-Dimethoxy-, 2,4-Dimethoxy- oder 3,4,5-Trimethoxyphenyl, Hydroxy-niederalkoxyphenyl, Hydroxy-diniederalkoxyphenyl, Niederalkanoylphenyl, Niederalkanoyloxyphenyl, Halogenphenyl, Nitrophenyl, Cyanphenyl, Amino-

oder Diaminophenyl, Amino-hydroxyphenyl, Niederalkylaminophenyl, Diniederalkylaminophenyl, Niederalkanoylaminophenyl, Sulfamoylphenyl, Halogenniederalkylphenyl oder Aminoniederalkylphenyl bedeuten, und ihren pharmazeutisch annehmbaren Salzen, dadurch gekennzeichnet, dass man die in Anspruch 1 genannten Massnahmen durchführt.

4. Verfahren zur Herstellung von Verbindungen gemäss Anspruch 3 der Formel I, worin X die Gruppe

‖
-C-H, Y Schwefel, A durch Niederalkoxyimino substituiertes Methylen, $R_1$ Wasserstoff, $R_2$ Wasserstoff oder Niederalkyl, und $R_3$ Furyl, Phenyl, Niederalkyl-, Diniederalkyl- oder Triniederalkylphenyl, Hydroxy- Dihydroxy- oder Trihydroxyphenyl, Hydroxy-niederalkylphenyl, Niederalkoxy-, Diniederalkoxy- oder Triniederalkoxyphenyl, Hydroxyniederalkoxyphenyl, Hydroxy-diniederalkoxyphenyl, Niederalkanoylphenyl, Niederalkanoyloxyphenyl, Halogenphenyl, Nitrophenyl, Cyanphenyl, Amino- oder Diaminophenyl, Amino-hydroxyphenyl, Niederalkylaminophenyl, Diniederalkylaminophenyl, Niederalkanoylaminophenyl, Halogenniederalkylphenyl oder Aminoniederalkylphenyl bedeuten, und ihren pharmazeutisch annehmbaren Salzen, dadurch gekennzeichnet, dass man die in Anspruch 1 genannten Massnahmen durchführt.

5. Verfahren zur Herstellung von Verbindungengemäss Anspruch 4 der Formel I, worin X die Gruppe

‖
-C-H, Y Schwefel, A durch Methoxyimino substituiertes Methylen, $R_1$ Wasserstoff, $R_2$ Wasserstoff oder Methyl und $R_3$ Fur-2- oder -3-yl, Phenyl, 2-, 3- oder 4-Methyl-, 2-, 3- oder 4-tert-Butyl-, 3,4-Dimethyl-, 2,3-Dimethyl-, 2,4-Dimethyl- oder 2,4,6-Trimethylphenyl, 4-Hydroxy-, 2,4-Dihydroxy-, 2,3,4-Trihydroxy-, 2,4,6-Trihydroxy- oder 3,4,5-Trihydroxyphenyl, 2-Hydroxy-3-methylphenyl, 4-Methoxy-, 3,4-Dimethoxy-, 2,3-Dimethoxy-, 2,4-Dimethoxy- oder 3,4,5-Trimethoxyphenyl, 4-Hydroxy-3-methoxyphenyl, 3-Hydroxy-2,4-dimethoxyphenyl, 2-, 3-oder 4-Acetylphenyl, 2-, 3- oder 4-Acetoxyphenyl, 2-, 3- oder 4-Fluor- oder 2-, 3- oder 4-Chlorphenyl, 2-, 3- oder 4-Nitrophenyl,

2-, 3- oder 4-Cyanphenyl, 2-, 3- oder 4-Aminophenyl oder 3,5-Diaminophenyl, 3-Amino-4-hydroxy- oder 4-Amino-2-hydroxyphenyl, 2- oder 4-Methylaminophenyl, 2-, 3- oder 4-Dimethylaminophenyl, 2-, 3- oder 4-Sulfamoylphenyl, 2-, 3- oder 4-Trifluormethylphenyl oder 2-, 3- oder 4-Aminomethylphenyl bedeuten, und ihren pharmazeutisch annehmbaren Salzen, dadurch gekennzeichnet, dass man die in Ansruch 1 genannten Massnahmen durchführt.

6. 7ß-[2-(2-Aminothiazol-4-yl)-2-Z-methoxyiminoacetamido]-3-cephem-4-carbonsäure-2-acetoxybenzoyloxymethylester gemäss Anspruch 5, dadurch gekennzeichnet, dass man die in Anspruch 1 genannten Massnahmen durchführt.

7. Verfahren zur Herstellung von 7ß-[2-(2-Aminothiazol-4-yl)-2-Z-methoxyiminoacetamido]-3-cephem-4-carbonsäure-4-methylbenzoyloxymethylester gemäss Anspruch 5, dadurch gekennzeichnet, dass man die in Anspruch 1 genannten Massnahmen durchführt.

8. Verfahren zur Herstellung von 7ß-[2-(2-Aminothiazol-4-yl)-2-Z-methoxyiminoacetamido]-3-cephem-4-carbonsäure-4-tert-butylbenzoyloxymethylester gemäss Anspruch 5, dadurch gekennzeichnet, dass man die in Anspruch 1 genannten Massnahmen durchführt.

9. Verfahren zur Herstellung von pharmazeutischen Präparaten enthaltend Verbindungen gemäss Anspruch 1 der Formel I, dadurch gekennzeichnet, dass man Verbindungen der Formel I oder pharmazeutisch verwendbare Salze davon nach dem Verfahren gemäss Anspruch 1 herstellt und diese mit einem pharmazeutischen Trägermaterial mischt.

10. Verfahren zur Herstellung von Verbindungen der Formel

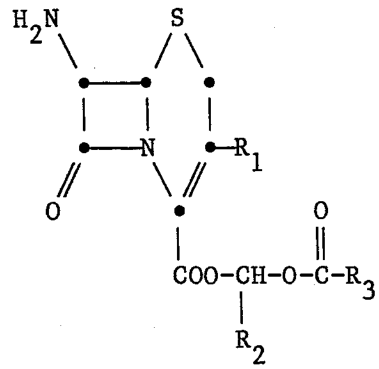

(II),

worin $R_1$, $R_2$ und $R_3$ die unter Formel I genannten Bedeutungen haben, dadurch gekennzeichnet, dass man eine Verbindung der Formel

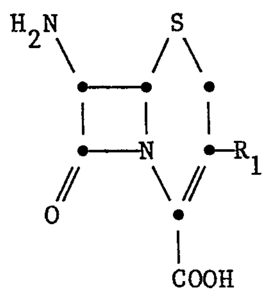

(XIV),

worin die 7ß-Aminogruppe gegebenenfalls geschützt ist und $R_1$ die unter Formel I genannte Bedeutung hat, oder ein reaktionsfähiges, funktionelles Derivat dieser Verbindung gegebenenfalls stufenweise mit einem die Gruppe

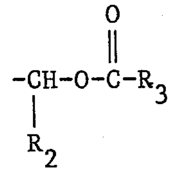

einführenden Veresterungsmittel umsetzt.

FO 7.4/RS/gs*